(19) **Europäisches Patentamt / European Patent Office / Office européen des brevets**

(11) **EP 3 521 817 B1**

(12) **EUROPEAN PATENT SPECIFICATION**

(45) Date of publication and mention of the grant of the patent:
**20.11.2024 Bulletin 2024/47**

(21) Application number: **17856478.7**

(22) Date of filing: **29.09.2017**

(51) International Patent Classification (IPC):
**G01N 27/416** (2006.01)   **A61F 13/84** (2006.01)
**B01J 20/26** (2006.01)   **G01N 31/00** (2006.01)
**G01N 33/44** (2006.01)

(52) Cooperative Patent Classification (CPC):
**A61F 13/84; B01J 20/26; G01N 31/00;
G01N 33/442;** G01N 27/4166

(86) International application number:
**PCT/JP2017/035642**

(87) International publication number:
**WO 2018/062531 (05.04.2018 Gazette 2018/14)**

(54) **METHOD FOR MEASURING CONTENT OF WATER-ABSORBING RESIN INCLUDED IN HYGIENIC MATERIAL, ABSORBENT ARTICLE, OR INTERMEDIATE PRODUCT IN MANUFACTURING PROCESS THEREOF, AND METHOD FOR MANUFACTURING HYGIENIC MATERIAL OR ABSORBENT ARTICLE USING SAME**

VERFAHREN ZUR MESSUNG DES GEHALTS AN WASSERABSORBIERENDEM HARZ IN EINEM HYGIENISCHEN MATERIAL, SAUGFÄHIGEN ARTIKEL ODER ZWISCHENPRODUKT IM HERSTELLUNGSVERFAHREN DAFÜR UND VERFAHREN ZUR HERSTELLUNG VON HYGIENISCHEM MATERIAL ODER EINES SAUGFÄHIGEN ARTIKELS DAMIT

PROCÉDÉ DE MESURE DE CONTENU DE RÉSINE ABSORBANT L'EAU COMPRIS DANS UN MATÉRIAU HYGIÉNIQUE OU ARTICLE ABSORBANT OU UN PRODUIT INTERMÉDIAIRE DANS SON PROCÉDÉ DE FABRICATION, ET PROCÉDÉ DE FABRICATION DE MATÉRIAU HYGIÉNIQUE OU D'ARTICLE ABSORBANT L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2016 JP 2016194387**

(43) Date of publication of application:
**07.08.2019 Bulletin 2019/32**

(73) Proprietor: **Nippon Shokubai Co., Ltd.
Osaka-shi, Osaka 541-0043 (JP)**

(72) Inventors:
• **KIMURA, Kazuki
Himeji-shi
Hyogo 671-1282 (JP)**
• **IWAMURA, Taku
Himeji-shi
Hyogo 671-1282 (JP)**

• **KITANO, Takahiro
Himeji-shi
Hyogo 671-1282 (JP)**
• **WADA, Hidenori
Himeji-shi
Hyogo 671-1282 (JP)**
• **FUJIMOTO, Taku
Himeji-shi
Hyogo 671-1282 (JP)**

(74) Representative: **Hoffmann Eitle
Patent- und Rechtsanwälte PartmbB
Arabellastraße 30
81925 München (DE)**

(56) References cited:
**JP-A- 2001 194 360    JP-A- 2014 110 845
JP-A- H01 260 361    JP-A- H09 243 622
JP-A- H11 506 209**

• **No further relevant documents disclosed**

**Description**

**TECHNICAL FIELD**

The present invention relates to a method for measuring an amount of a water-absorbing resin included in a hygienic material, an absorbent article, or an intermediate product in a manufacturing process thereof and a method for manufacturing a hygienic material or an absorbent article using the same.

**BACKGROUND ART**

In recent years, a water-absorbing resin has been widely used as one of constituent materials of hygienic materials or absorbent articles of paper diapers, sanitary napkins, incontinence pads, and the like, for the purpose of absorbing the body fluid. Since the absorption performance of such hygienic materials or absorbent articles is determined depending on an amount of the water-absorbing resin to be contained, in product development, quality management, and performance evaluation, it is indispensable to measure a content of the water-absorbing resin contained per one sheet of the hygienic material or the absorbent article, or contained in an intermediate product in a manufacturing process thereof.

As the measuring method, JP 1-260361 A discloses a method for measuring an amount of an alkali metal ion isolated from a water-absorbing resin after a water-absorbing material including the water-absorbing resin and other materials is processed with a strong acid solution, and measuring the amount of the water-absorbing resin in the water-absorbing material.

Further, JP 9-243622 A discloses a method for measuring an amount of a water-absorbing resin in an absorber by processing the water-absorbing resin in the absorber with a strong acid solution including a known amount of hydrogen ions and then quantitatively determining a surplus hydrogen ion excluding a hydrogen ion which is consumed by being substituted with an alkali metal salt of an alkali metal base constituting the water-absorbing resin.

Furthermore, JP 2001-194360 A discloses a method for measuring an amount of a water-absorbing resin in an absorber by processing the absorber including the water-absorbing resin with an aqueous solution including a neutral salt to obtain a hydrogel dispersion liquid, and then titrating the hydrogel dispersion liquid using a basic solution or an acidic solution.

**SUMMARY OF INVENTION**

Technical Problem

However, in the methods described in the above-described respective Patent Literatures, the strong acid processing of the water-absorbing material and the titration with an acidic solution or a basic solution are essential and it is necessary to use a reagent harmful to a human body, so that there is a restriction that only persons having advanced knowledge of chemical products handle those methods.

Therefore, an object of the present invention is to provide a convenient, safe, and highly precise method for measuring an amount of a water-absorbing resin in a hygienic material, an absorbent article, or an intermediate product in a manufacturing process thereof, which solves the above-described problems.

Solution to Problem

The present inventors have conducted intensive studies in order to solve the above-described problems. As a result, the present inventors have found that an amount of a water-absorbing resin can be measured with high precision by pre-processing a water-absorbing resin with an aqueous solution including a total ionic strength adjusting agent without being isolated from a hygienic material, an absorbent article, or an intermediate product in a manufacturing process thereof including a water-absorbing resin which has a water-soluble cation in the form of an ion-dissociating group, measuring an amount of a predetermined water-soluble cation in the pre-processed supernatant liquid by an ion selective electrode method, and estimating an amount of the water-absorbing resin in the hygienic material, the absorbent article, or the intermediate product in the manufacturing process thereof by a calibration curve which has been created in advance, thereby completing the present invention.

**BRIEF DESCRIPTION OF DRAWINGS**

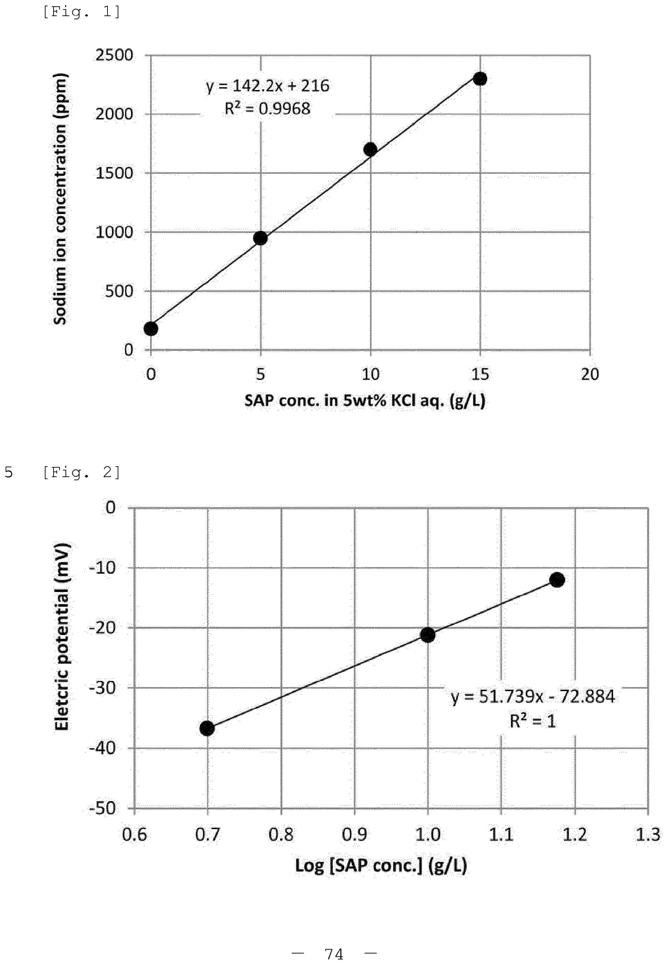

Fig. 1 is a graph in which results of a sodium ion concentration (ppm) (longitudinal axis) of a supernatant liquid with respect to a water-absorbing resin weight (concentration: g/L) (horizontal axis) per 1 L of 5% by weight potassium chloride aqueous solution obtained in Example 1 are plotted. Incidentally, in Fig. 1, a calibration curve obtained by the least-square method (primary approximation formula) is shown along with a determination coefficient $R^2$ ($R^2$ = 0.9968) thereof.

Fig. 2 is a graph in which results of an electrode potential (mV) (longitudinal axis) with respect to a value of common logarithm (log[SAP conc.]) (horizontal axis) of a water-absorbing resin weight (concentration: SAP conc. [g/L]) per 1 L of 5% by weight potassium chloride aqueous solution obtained in Example 2 are plotted. Incidentally, in Fig. 2, a calibration curve obtained by the least-square method (primary approximation formula) is shown along with a determination coefficient ($R^2$ = 1) thereof.

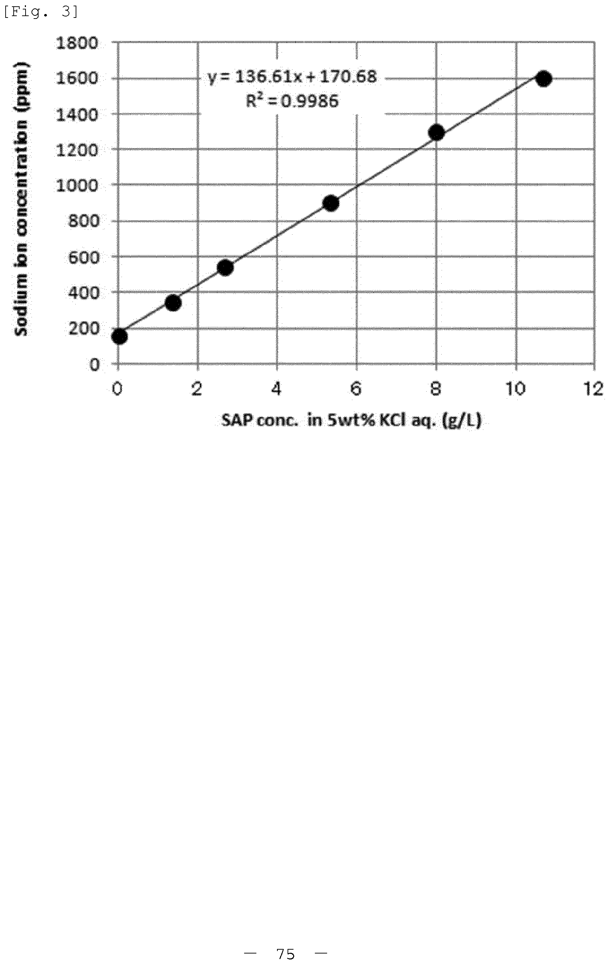

Fig. 3 is a graph in which results of a sodium ion concentration (ppm) (longitudinal axis) of a supernatant liquid with respect to a water-absorbing resin weight (concentration: g/L) (horizontal axis) per 1 L of 5% by weight potassium chloride aqueous solution obtained in Example 4 are plotted. Incidentally, in Fig. 3, a calibration curve obtained by the least-square method

(primary approximation formula) is shown along with a determination coefficient $R^2$ ($R^2$ = 0.9986) thereof.

## DESCRIPTION OF EMBODIMENTS

An aspect of the present invention relates to a method for measuring an amount of a water-absorbing resin included in a hygienic material, an absorbent article, or an intermediate product in a manufacturing process thereof, wherein the water-absorbing resin includes a water-soluble cation derived from neutralization of an acid group of the water-absorbing resin. The measuring method includes:

A) a step for measuring an amount of a predetermined water-soluble cation in a reference sample including a known amount of the water-absorbing resin and creating a calibration curve indicating a relation between the amount of the water-absorbing resin and the amount of the predetermined water-soluble cation in the reference sample on the basis of the obtained measurement result;

B) a step for measuring an amount of the predetermined water-soluble cation in a measurement sample, which includes a portion including the water-absorbing resin, of the hygienic material, the absorbent article, or the intermediate product in the manufacturing process thereof; and

C) a step for calculating an amount of the water-absorbing resin in the measurement sample by the calibration curve created in the step A) from the amount of the predetermined water-soluble cation measured in the step B),

Further, the step A) and the step B) include measuring the amount of the predetermined water-soluble cation using an ion selective electrode method and including the following steps M1) and M2):

M1) a step for immersing a sample in an aqueous solution including a total ionic strength adjusting agent (pre-processing step); and

M2) a step for measuring the amount of the predetermined water-soluble cation in a pre-processed supernatant liquid obtained in the step M1). The water-soluble cation is selected from a sodium ion, a potassium ion and an ammonium ion, and the total ionic strength adjusting agent is selected from a potassium salt, a calcium salt, a magnesium salt and an ammonium salt when the water-soluble cation is a sodium ion; the total ionic strength adjusting agent is selected from a sodium salt, a calcium salt, a magnesium salt and an ammonium salt when the water-soluble cation is a potassium ion; and the total ionic strength adjusting agent is selected from a sodium salt, a potassium salt, a calcium salt and a magnesium salt when the water-soluble cation is an ammonium ion

By the method for measuring an amount of a water-absorbing resin in a hygienic material, an absorbent article, or an intermediate product in a manufacturing process thereof according to the present invention, it is possible to measure the content of the water-absorbing resin without using a strong acid, it is not necessary to perform titration using an acidic solution or a basic solution, and it is possible to perform a quantitative determination operation of the water-absorbing resin only by a safer reagent. Incidentally, the measurement of the amount of the predetermined water-soluble cation including the steps M1) and M2) is performed in both the step A) and the step B). Therefore, each of the step M1) and the step M2) corresponding thereto is performed in both the step A) and the step B).

Hereinafter, the present invention will be described in detail.

As described above, an aspect of the present invention relates to a method for measuring an amount of a water-absorbing resin included in a hygienic material, an absorbent article, or an intermediate product in a manufacturing process thereof, wherein the water-absorbing resin includes a water-soluble cation derived from neutralization of an acid group of the water-absorbing resin.

The term "water-absorbing resin" indicates a water-swelling and water-insoluble polymer gelatinizer and refers to one satisfying the following physical properties. That is, the water-absorbing resin indicates a polymer gelatinizer satisfying the physical properties that CRC defined by ERT441.2-02, as "water-swelling", is 5 g/g or more and Ext defined by ERT470.2-02, as "water-insoluble", is 50% by weight or less.

The water-absorbing resin can be appropriately designed depending on use application thereof, and although not particularly limited, the water-absorbing resin is preferably a hydrophilic crosslinked polymer which is obtained by crosslinking polymerization and has a hydrophilic group such as a carboxyl group, a sulfonic acid group, a phosphoric acid group, a tertiary amino group, a quaternary ammonium group, a hydroxyl group, or a polyethylene oxide group, as a constituent unit thereof. The hydrophilic crosslinked polymer may be or may not be subjected to surface-crosslinking (also known as post-crosslinking or secondary crosslinking) in addition to the internal crosslinking, but from the viewpoint of the physical properties, a water-absorbing resin subjected to surface-crosslinking is preferable. These water-absorbing resins preferably have a water-soluble cation as an ion-dissociating group and have a carboxylic group, a sulfonic acid group, or a phosphoric acid group in terms of a quantitative determination target, and are particularly preferably a carboxylic group. Herein, a polyacrylic acid salt is most preferable. The form of these water-absorbing resins is not particularly limited, and may be any of a powder form (also known as a particle form), a sheet form, a fiber form, a film form, a gel form, and a dispersion liquid (water-absorbing resin emulsion), but a powder form (also known as a particle form) is preferable from the viewpoint of applying the measuring method according to the present invention. Incidentally, in the case of a powder form, the particle size of the water-absorbing resin is appropriately designed depending on use application thereof. Regarding the particle size distribution in the case of using the water-absorbing resin in a hygienic material or an absorbent article, particles of 10 to 2000 $\mu$m, 100 to 1000 $\mu$m, or 150 to 850 $\mu$m are used as main components (particularly, 90% by weight or more, and 95% by weight or more). Further, the ratio of particles having a particle size of less than 150 $\mu$m is preferably 10% by mass or less. Regarding the method for measuring the particle size distribution (upper and lower limits), the particle size distribution is measured using a standard sieve according to the method disclosed in US 7638570 or EDANA ERT420.2-02.

Further, the water-absorbing resin which has a water-soluble cation (preferably an alkali metal cation described later) as an ion-dissociating group is partially or completely neutralized, and the neutralization rate thereof (molar ratio of neutralized acid group in the whole acid group) may be more than 100% and is in a range of preferably 10 to 100 mol%, and further preferably 30 to 95%, 40 to 90 mol%, or 50 to 80 mol%. With such a neutralization rate, a high content of the alkali metal cation is achieved in addition to a high water absorption capacity and a high water absorption speed, and the content of the water-absorbing resin can be more stably measured. Further, in the water-absorbing resin of the present invention, a water-soluble cation derived from neutralization of the acid group in the polymer main chain (for example, about 1.94 g of sodium cation derived from COO-Na is contained in 10 g (solid content 100%) of partially neutralized sodium polyacrylate having a neutralization rate of 75 mol%, but, as auxiliary materials other than a monomer of the water-absorbing resin, a cation (for example, COO-Na in an anionic surfactant), various organic acid salts (for example; Na lactate and Na citrate), a chelating agent (for example; a neutralized salt of a phosphoric acid-based or aminocarboxylic acid-based chelating agent, for example, 4 Na of EDTA or the like)) may be contained (for example, 0.0001 to 5% by weight of the entirety), and in the present invention, those auxiliary materials are included as the water-absorbing resin and the method for measuring a water-absorbing resin of the present invention can be applied.

Further, the acid group of the water-absorbing resin is partially or completely neutralized with the water-soluble cation. According to this, the water-absorbing resin has the water-soluble cation in the form of the ion-dissociating group. In the measuring method according to the present invention, the amount of the water-absorbing resin is measured by quantitative determination of the water-soluble cation. The water-soluble cation is selected from a sodium ion, a potassium ion and an ammonium. Preferably, the water-soluble cation is a sodium ion.

Further, the water-absorbing resin is not limited to the case of the whole amount (100% by weight) being a polymer, and the water-absorbing resin may be a water-absorbing resin composition including an additive or the like in a range satisfying the above-mentioned physical properties (CRC and Ext). Furthermore, the form of the water-absorbing resin may be any of powder, a particle form, a fiber form, and the like and there is no limitation. In a case where the form of the water-absorbing resin is powder or a particle form, the surface thereof may be subjected to a surface crosslinking treatment by a conventionally known surface cross-linking agent (for example, polyhydric alcohol or the like).

The water-absorbing material including the absorbent resin which has the water-soluble cation in the form of the ion-dissociating group is also generally called as "absorber (absorbent core)" and specifically, for example, the water-absorbing material exists between a liquid permeable top sheet and a liquid impermeable back sheet joined to the top sheet in a hygienic material such as a paper diaper, a sanitary napkin and a so-called incontinence pad, and serves a function of absorbing urine, body fluid, or the like discharged from the body of a wearer. In general, examples of the water-absorbing material (or the absorber) including the absorbent resin which has the ion-dissociating group include a mixture in which a water-absorbing resin is dispersed in natural fibers such as cellulose fibers and wood-crushed pulp or synthetic fibers such as cellulose acetate and rayon, and a complex of fiber-water-absorbing resin obtained by immersing a monomer aqueous solution as a raw material of the water-absorbing resin in a fiber base material such as a water-absorbing resin sheet or a non-woven fabric in which the water-absorbing resin is sandwiched by two sheets of upper and lower non-woven fabrics and fixed and polymerizing the obtained product, but the measurement target of the present invention is not particularly limited to these examples. Further, there have been those having a multi-layered structure in which a water-absorbing material (or an absorber) including an absorbent resin which has an ion-dissociating group is separated into an upper layer and a lower layer among the hygienic materials which are marketed in recent years, but those may also be a measurement target of the present invention. Incidentally, the concept of "the water-absorbing resin, which has a water-soluble cation in the form of an ion-dissociating group, included in the hygienic material, the absorbent article, or the intermediate product of the manufacturing process thereof" also includes the "water-absorbing resin" itself. That is, it is obvious that the method for measuring a water-absorbing resin according to the present invention can also be executed using the "water-absorbing resin which has a water-soluble cation in the form of an ion-dissociating group" itself as the measurement target.

Hereinafter, the present invention will be described in more detail in order of the steps.

· Step A)

· In the step A), an amount of a predetermined water-soluble cation in a reference sample including a known amount of the water-absorbing resin included in the hygienic material, the absorbent article, or the intermediate product in the manufacturing process thereof is measured, and a calibration curve indicating a relation between the amount of the water-absorbing resin and the amount of the predetermined water-soluble cation in the reference sample is created on the basis of the obtained measurement result.

(Reference Sample)

In the step A), first, the reference sample used in the measurement for creating the calibration curve is prepared. Herein, when a water-absorbing resin, which is a measurement target, before being incorporated into the hygienic material, the absorbent article, or the intermediate product in the manufacturing process thereof is available (for example, in the case of applying the measuring method of the present invention as the process management in the manufacturing line for paper diapers, or the like), the water-absorbing resin may be used as a reference sample for creating a calibration curve without any change since the water-absorbing resin for being used as the reference sample can be easily obtained in the form of a raw material.

On the other hand, when a water-absorbing resin, which is a measurement target, before being incorporated into the hygienic material, the absorbent article, or the intermediate product in the manufacturing process thereof is not available (for example, in the case of applying the method according to the present invention in order to measure a content of a water-absorbing resin included in a commercially available hygienic material, an commercially available absorbent article, or an intermediate product in a manufacturing process thereof), it is preferable that the step A) further includes a step A1) for collecting the water-absorbing resin from the hygienic material, the absorbent article, or the intermediate product in the manufacturing process thereof before the step M1) in the step A). Further, it is preferable that the water-absorbing resin collected in the step A1) is used as the reference sample for creating a calibration curve. With such an embodiment, even in a case where the water-absorbing resin as a raw material is not available (in a state where the water-absorbing resin is not incorporated into the hygienic material, the absorbent article, or the intermediate product in the manufacturing process thereof), it is possible to measure the content of the water-absorbing resin with high precision.

At this time, in the collecting of the water-absorbing resin from the hygienic material, the absorbent article, or the intermediate product in the manufacturing process thereof, the hygienic material, the absorbent article, or the intermediate product in the manufacturing process thereof as the measurement target is prepared, an absorber is extracted therefrom, and the water-absorbing resin is collected from the absorber. Herein, the method for collecting the water-absorbing resin is not particularly limited, but it is preferable that constituents of the absorber other than the water-absorbing resin (for example, cellulose, wood-crushed pulp, and the like) are not mixed in the collected water-absorbing resin as much as possible. As the method for removing constituents other than the water-absorbing resin as much as possible, in addition to classification with a sieve using a difference in size between the water-absorbing resin and constituents other than the water-absorbing resin, gravity separation using a difference in specific gravity, wind force separation, and the like are exemplified, and separation can also be performed in combination of a plurality of those methods.

Further, since the water-absorbing resin in a dry state has high fluidity and is easily separated, an environment in extraction operation is preferably an environment in which temperature and humidity are adjusted as much as possible.

Such an environment may be generally in a range of a temperature of 20 to 35°C and a relative humidity of 30 to 60%. Even in a case where a water-absorbing resin before being incorporated into the hygienic material, the absorbent article, or the intermediate product in the manufacturing process thereof as the measurement target is available, the environment when the water-absorbing resin as the reference sample is stored before measurement is preferably the environment of temperature and humidity described above.

Incidentally, in the step for collecting the water-absorbing resin for creating a calibration curve, it is possible to figure out an approximate amount of the water-absorbing resin included in each hygienic material, absorbent article, or intermediate product in the manufacturing process thereof as a predicted weight. Therefore, in a preferred embodiment of the measuring method according to the present invention, the step A) further includes a step A2) for measuring an approximate amount of the water-absorbing resin contained in the hygienic material, the absorbent article, or the intermediate product in the manufacturing process thereof when the water-absorbing resin is collected from the hygienic material, the absorbent article, or the intermediate product in the manufacturing process thereof in the step A1), and determining a range of the amount of the water-absorbing resin in the calibration curve to be created on the basis of the obtained measurement result. For example, in a case where a predicted weight of the water-absorbing resin included in each hygienic material/absorbent article/intermediate product in the manufacturing process thereof is 10 g and the water-absorbing resin is dispersed in 500 mL of the total ionic strength adjusting agent aqueous solution, the upper limit and the lower limit of the water-absorbing resin concentration in the total ionic strength adjusting agent aqueous solution of the calibration curve created in the step A) can be set to 50 g/L and 1 g/L, respectively. In this way, the upper limit and the lower limit of the calibration curve range is preferably created with the predicted weight sandwiched therebetween (preferably, such that the predicted weight is close to the center in the range). With such an embodiment, it is possible to improve the reliability of the calibration curve near the predicted weight and it is possible to measure the content of the water-absorbing resin with higher precision.

Incidentally, the reference sample is used in the creation of the calibration curve through the measuring of the amount of the water-soluble cation described later. Therefore, it is necessary to measure the amount of the water-absorbing resin constituting the reference sample by weighing or the like.

(Measurement of Amount of Water-Soluble Cation in Reference Sample)

In the step A), the amount of the water-soluble cation in (the known amount of) the reference sample prepared above is measured. Specifically, the amount of the predetermined water-soluble cation in the reference sample including the known amount of the water-absorbing resin is measured. Herein, in response to the fact that it is necessary to determine a water-soluble cation in advance as the measurement target in the measurement of the amount of the water-soluble cation by an ion selective electrode method described later in a case where the water-absorbing resin included in the reference sample includes a plurality of water-soluble cations, the expression "the amount of the predetermined water-soluble cation is measured" means that the amount of the "water-soluble cation determined in advance" is measured. Incidentally, the predetermined water-soluble cation may be one kind alone or two or more kinds, but from the convenience of measurement, the predetermined water-soluble cation is preferably one kind alone.

As described above, the step A) includes measuring the amount of the predetermined water-soluble cation using an ion selective electrode method. Further, the step A) includes the following steps M1) and M2):

M1) a step for immersing a sample in an aqueous solution including a total ionic strength adjusting agent (pre-processing step); and
M2) a step for measuring the amount of the predetermined water-soluble cation in a pre-processed supernatant liquid obtained in the step M1).

(Pre-Processing Step)

In the pre-processing step M1) in the step A), (the known amount of) the reference sample prepared above is immersed in an aqueous solution including a total ionic strength adjusting agent (in the present specification, also referred to as the "total ionic strength adjusting agent aqueous solution"). According to the pre-processing step M1), the water-soluble cation derived from the ion-dissociating group of the water-absorbing resin included in the reference sample can be dispersed in the supernatant liquid of the total ionic strength adjusting agent aqueous solution, and the amount of the water-soluble cation can be measured by the ion selective electrode method in the step M2) described later.

In the present invention, the total ionic strength adjusting agent used in the pre-processing step M1) does not hinder the measurement of the water-soluble cation serving as the measurement target.

In a case where the amount of the sodium ion as the water-soluble cation is measured, the total ionic strength adjusting agent is selected from a potassium salt, a calcium salt, a magnesium salt and an ammonium salt of a strong electrolyte, and examples thereof include potassium chloride, potassium sulfate, potassium nitrate, potassium hydroxide, calcium chloride, calcium sulfate, calcium nitrate, calcium hydroxide, magnesium chloride, magnesium sulfate, magnesium nitrate, magnesium hydroxide, ammonium chloride, ammonium sulfate, ammonium nitrate, ammonium hydroxide, and the like. Of them, from the viewpoint of availability, high solubility to water, and safety of a material, potassium chloride or ammonium chloride is preferable and potassium chloride is particularly preferable. Further, in a case where the amount of a potassium ion as the water-soluble cation is measured, the total ionic strength adjusting agent is selected from a sodium salt, a calcium salt, a magnesium salt and an ammonium salt of a strong electrolyte, and examples thereof include sodium chloride, sodium sulfate, sodium nitrate, sodium hydroxide, calcium chloride, calcium sulfate, calcium nitrate, calcium hydroxide, magnesium chloride, magnesium sulfate, magnesium nitrate, magnesium hydroxide, ammonium chloride, ammonium sulfate, ammonium nitrate, ammonium hydroxide, and the like. Of them, from the viewpoint of availability, high solubility to water, and safety of a material, sodium chloride or ammonium chloride is preferable. Furthermore, in a case where the amount of an ammonium ion as the water-soluble cation is measured, the total ionic strength adjusting agent is selected from a sodium salt, a potassium salt, a calcium salt and a magnesium salt of a strong electrolyte, and examples thereof include sodium chloride, sodium sulfate, sodium nitrate, sodium hydroxide, potassium chloride, potassium sulfate, potassium nitrate, potassium hydroxide, calcium chloride, calcium sulfate, calcium nitrate, calcium hydroxide, magnesium chloride, magnesium sulfate, magnesium nitrate, magnesium hydroxide, and the like. Of them, from the viewpoint of availability, high solubility to water, and safety of a material, sodium chloride or potassium chloride is preferable.

As described above, the type of the total ionic strength adjusting agent can be appropriately selected, but it is preferable to further include a step N) for selecting a total ionic strength adjusting agent to be used depending on the type of the water-soluble cation to be measured by the ion selective electrode method in the step M2) described later. Further, when the type of the water-soluble cation of the water-absorbing resin included in the reference sample is not known, it is preferable to further include a step L) for determining the type of the water-soluble cation, before the pre-processing step M1) in the step A). The method for determining the type of the water-soluble cation of the water-absorbing resin is not particularly limited, but examples of easiest methods include a method for confirming the type of the water-soluble cation with safety data sheet (SDS) of the water-absorbing resin to be used, and a method for confirming the type of the water-soluble cation from a raw material supplier. Incidentally, in a case where the measurement target is an unknown water-absorbing resin, an ICP (emission spectroscopic analysis method) or the like may be used.

In the present invention, the total ionic strength adjusting agent used in the measurement of the water-absorbing resin is used in the form of an aqueous solution. The concentration of the total ionic strength adjusting agent in the aqueous solution is not particularly limited as long as it is adjusted to be a sufficiently higher ionic strength than the concentration of the water-soluble cation as the measurement target in the sample solution, but is generally in a range of 0.05 to 20 mol/L and preferably in a range of 0.1 to 10 mol/L.

Further, the pH of the total ionic strength adjusting agent aqueous solution used in the measurement of the water-absorbing resin in the present invention is not particularly limited since the pH thereof depends on the type and the amount of the total ionic strength adjusting agent to be used, but is usually 2 to 12, preferably 3 to 11, and further preferably 4 to 10.

In the present invention, a container holding the total ionic strength adjusting agent aqueous solution is not particularly limited, but is preferably a container that does not inhibit the measurement using the ion selective electrode. Further, as prior confirmation, it is preferable to confirm whether the measurement value is unstable by a blank test. For example, when a plastic jug, cup or the like which is sufficiently washed is used, the influence of ions derived from a container is easily excluded, which is more preferable.

In the pre-processing step M1) of the step A), the reference sample is immersed in the total ionic strength adjusting agent aqueous solution and dispersed for a predetermined time. The amount of the total ionic strength adjusting agent aqueous solution in which the reference sample is dispersed in the pre-processing step M1) may be appropriately selected depending on the amount of the reference sample and is not particularly limited. For example, it is sufficient that the total ionic strength adjusting agent aqueous solution per one measurement is 1000 mL. Further, the concentration of the water-absorbing resin included in the reference sample of the total ionic strength adjusting agent aqueous solution is preferably 1 g/L or more, and the upper limit may be 100 g/L or less. In order to obtain a stable measurement result, it is preferable to perform stirring during dispersing, but stirring is not essential, and it is also sufficient to perform stirring only at the final stage after a predetermined time elapses. In a preferred embodiment, when the reference sample is pre-processed with the aqueous solution including the total ionic strength adjusting agent, stirring is performed for at least one second or longer.

(Measuring Step)

In the step M2) in the step A), the amount of the predetermined water-soluble cation in a pre-processed supernatant liquid obtained in the aforementioned pre-processing step M1) is measured (measuring step).

Specifically, for example, the total ionic strength adjusting agent aqueous solution in the aforementioned pre-processing step M1) is left to stand still for a predetermined time and then filtered, the supernatant liquid is then collected, and the concentration of the predetermined water-soluble cation as the measurement target is measured using the ion selective electrode which has been corrected in advance.

The ion selective electrode used in the measurement of the content of the water-absorbing resin in the pre-processing step M2) is not particularly limited, but may be an electrode that is generally marketed and reacts with a specific ion in the aqueous solution to generate a potential corresponding to the concentration (activity) thereof.

For example, in the case of measuring the amount of sodium ion as the water-soluble cation, examples of the electrode include a sodium ion selective electrode CI-6734 manufactured by SHIMADZU RIKA CORPORATION, a sodium composite electrode ORION 8611BNWP manufactured by Thermo Fisher Scientific Inc., a sodium ion electrode 1512A-10C and a compact sodium ion meter B-722 manufactured by HORIBA, Ltd, and the like. Further, in the case of measuring the amount of potassium ion as an alkali metal ion, examples of the electrode include a potassium ion selective electrode CI-6733 manufactured by SHIMADZU RIKA CORPORATION, a potassium composite electrode ORION 9719BNWP manufactured by Thermo Fisher Scientific Inc., a potassium ion electrode (composite type) 6582-10C and a compact potassium ion meter B-731 manufactured by HORIBA, Ltd, and the like. Further, in the case of measuring the amount of ammonium ion as the water-soluble cation, examples of the electrode include an ammonium ion selective electrode CI-6717 manufactured by SHIMADZU RIKA CORPORATION, an ammonia composite electrode ORION 9512HPBNWP manufactured by Thermo Fisher Scientific Inc., an ammonia electrode (composite type) 5002A-10C manufactured by HORIBA, Ltd, and the like.

Subsequently, the calibration curve is created from the measurement value obtained above and the weight of the reference sample used in the measurement. When the calibration curve is created, the calibration curve may be created by using a plot of the measurement value of the amount of the predetermined water-soluble cation in one reference sample, which is arbitrarily selected, corresponding to the amount of the water-absorbing resin and a plot of the measurement value of the amount of the predetermined water-soluble cation in a reference sample not using a water-absorbing resin (concentration of 0 g/L), but when the calibration curve is created using preferably two or more reference samples other than the reference sample not using a water-absorbing resin and more preferably three or more reference samples other than the reference sample not using a water-absorbing resin, the calibration curve with higher precision is obtained. Incidentally, in a case where the calibration curve to be created uses Nernst's equation described later, since a value of common logarithm of the concentration [SAP conc.] in the total ionic strength adjusting agent aqueous solution of the water-absorbing resin is used, the measurement value of the amount of the predetermined water-soluble cation in the reference sample not using a water-absorbing resin (concentration of 0 g/L) cannot be plotted, and when the calibration curve is created using essentially two or more, preferably three or more reference samples, the calibration curve with high precision is obtained.

Depending on the type of the ion selective electrode to be used in measurement, the amount of the water-soluble cation of the measurement target existing in the supernatant liquid of the total ionic strength adjusting agent aqueous solution is measured as an ion concentration (the unit is, for example, ppm) or as an electrode potential (the unit is, for example, mV) that is an original electrochemical measurement result of the electrode.

Of them, in a case where the amount of the water-soluble cation of the measurement target existing in the supernatant liquid is measured as an ion concentration, the concentration of the water-absorbing resin derived from the reference sample in the total ionic strength adjusting agent aqueous solution is plotted on the horizontal axis and the concentration (measurement value) of the predetermined water-soluble cation of the measurement target is plotted on the longitudinal axis, so that a calibration curve can be created as a primary approximation formula.

On the other hand, in a case where the amount of the water-soluble cation of the measurement target existing in the supernatant liquid is measured as an electrode potential, a calibration curve can be created by applying Nernst's equation (Equation 1).

$$E = E_0 + \alpha \cdot \log C \qquad (\text{Equation 1})$$

In the equation,

E represents an electrode potential (mV) and a potential measured in a liquid to be measured;
$E_0$ represents a reference potential (mV) and a constant determined in a measurement system;
$\alpha$ represents a proportionality coefficient and an inclination of a calibration curve; and
C represents an ionic activity in the solution.

Further, the ionic activity C is proportional to a concentration of the water-absorbing resin in the total ionic strength adjusting agent aqueous solution included in the reference sample as the measurement target in the present invention [SAP conc.], and thus the following equation (Equation 2) is established using an activity coefficient $\beta$.

$$C = \beta \cdot [\text{SAP conc.}] \qquad (\text{Equation 2})$$

As a result, Equation 1 can be changed from Equation 2 to the following equation (Equation 3).

$$E = \gamma + \delta \cdot \log[\text{SAP conc.}] \qquad (\text{Equation 3})$$

Therefore, log[SAP conc.] is plotted on the horizontal axis and the electrode potential is plotted on the longitudinal axis, so that a calibration curve can be created as a primary approximation formula.

Incidentally, the conversion formula of the calibration curve is obtained by an approximation method such as the least-square method. In the case of obtaining the conversion formula of the calibration curve using the least-square method, since a larger determination coefficient $R^2$ of the approximation formula representing the precision of the calibration curve is preferable, a predetermined threshold is determined with respect to the determination coefficient $R^2$ according to the precision required for the calibration curve, and in a case where the determination coefficient $R^2$ is the predetermined threshold or more, the step may proceed to the subsequent step. In other words, in a case where the determination coefficient $R^2$ of the obtained approximation formula of the calibration curve is less than the predetermined threshold, it is preferable that the creation of the calibration curve is repeatedly performed until the value of the determination coefficient $R^2$ becomes the predetermined threshold or more. In a case where the determination coefficient $R^2$ of the approximation formula of the calibration curve is less than the predetermined threshold, the reason for this is considered that the type and the concentration of the total ionic strength adjusting agent to be used with respect to the water-absorbing resin as the measurement target are not preferable, and the like. Therefore, in a case where the creation of the calibration curve is repeated, the creation thereof is preferably performed while those conditions are appropriately adjusted. Incidentally, the predetermined threshold can be set, for example, to 0.9.

· Step B)

In the step B), the amount of the predetermined water-soluble cation in a measurement sample, which includes a portion including the water-absorbing resin, of the hygienic material, the absorbent article, or the intermediate product in the manufacturing process thereof, is measured.

In this way, the measurement sample used in the step B) includes a portion, which includes the water-absorbing resin, of the hygienic material, the absorbent article, or the intermediate product in the manufacturing process thereof. That is, the pre-processing step M1) and the measuring step M2) are performed similarly to the measurement of the amount of the predetermined water-soluble cation in the aforementioned step A), except that the measurement sample is set as a target. Incidentally, since the measurement sample is used in the measurement of the amount of the water-absorbing resin included in the hygienic material, the absorbent article, or the intermediate product in the manufacturing process thereof, the measurement sample preferably includes "the total amount of the water-absorbing resin" for the purpose of quality management in the manufacturing process, but a part of the water-absorbing resin may be included, or a portion including a desired amount of the water-absorbing resin is collected from the water-absorbing resin included in the intermediate in the manufacturing process and the content thereof may be measured. In a case where the amount of a part of the water-absorbing resin is measured, there is a method for calculating the amount of the water-absorbing resin included in the whole absorber from the area ratio of the water-absorbing resin to be measured to the whole absorber, but the method is not limited thereto. Further, the concept of "the portion, which includes the water-absorbing resin, of the hygienic material, the absorbent article, or the intermediate product in the manufacturing process thereof" also includes a case where the entirety of the hygienic material, the absorbent article (for example, a paper diaper, a sanitary napkin, an incontinence pad, or the like), or the intermediate (for example, an absorbent core or a water-absorbing resin sheet) is used without any changes. Furthermore, the portion may be a portion of the entirety obtained by cutting the hygienic material, the absorbent article, or the intermediate product in the manufacturing process thereof as necessary.

However, for the purpose of quality management in the manufacturing process, it is preferable to use a part including the water-absorbing resin as a target of the quantitative determination as the measurement sample, and particularly, it is preferable to extract an absorber from the hygienic material, the absorbent article, or the intermediate product in the manufacturing process thereof and then use the absorber as the measurement sample.

In a preferred embodiment, it is preferable that the step B) further includes a step B1) for collecting the portion including the water-absorbing resin from the hygienic material, the absorbent article, or the intermediate product in the manufacturing process thereof before the pre-processing step M1) in the step B), and the portion including the water-absorbing resin collected in the step B1) is used as the measurement sample.

Herein, in general, since the hygienic material (or the absorbent article) uses many constituent materials such as a waist belt, a side gather, a leg cuff, a pressure-sensitive adhesive tape, a surface fastener called hook and loop, and rubber in addition to the aforementioned top sheet and back sheet, it is possible to reduce bulkiness of the measurement sample to be immersed in the total ionic strength adjusting agent aqueous solution in the pre-processing step M1) of the step B) by excluding constituent materials not containing the water-absorbing resin. Therefore, it is preferable that the absorber can be separated from the hygienic material/the absorbent article, but depending on a product, there is also a case where the water-absorbing resin and another constituent material are integrally molded with a glue or the like, so that complete separation is not essential. In order not to scatter the water-absorbing resin as the measurement target outside the measurement system, those from which unnecessary other constituent materials such as a waist belt, a side gather, a leg cuff, a pressure-sensitive adhesive tape, a surface fastener called hook and loop, and rubber are removed in a possible range may be used as the measurement sample including the absorber.

Similarly to the pre-processing step M1) using the reference sample as a target in the aforementioned step A), the measurement sample is immersed in the total ionic strength adjusting agent aqueous solution and dispersed for a predetermined time in the pre-processing step M1) using the measurement sample as a target in the step B). The amount of the total ionic strength adjusting agent aqueous solution in which the measurement sample is dispersed in the pre-processing step M1) of the step B) may be appropriately selected depending on the amount of the measurement sample (for example, the hygienic material, the absorbent article, or the intermediate product in the manufacturing process thereof or the absorber extracted therefrom) and is not particularly limited. For example, it is usually sufficient that the total ionic strength adjusting agent aqueous solution per one measurement is only 5000 mL or less. In order to obtain a stable measurement result, it is preferable to perform stirring during dispersing, but stirring is not essential, and it is sufficient to perform stirring only at the final stage after a predetermined time elapses. In a preferred embodiment, when the reference sample is pre-processed with the aqueous solution including the total ionic strength adjusting agent, stirring is performed for at least one second or longer. Similarly, in order to obtain a stable quantitative determination result, it is preferable that the predetermined dispersing time and a predetermined still-standing time immediately before the filtration of the supernatant liquid in the pre-processing step M1) of the step B) are set to be values close to the dispersing time at the time of creating the calibration curve and a predetermined still-standing time immediately before the filtration of the supernatant liquid in the step A), and a difference therebetween is set preferably within 5 minutes and further preferably within 1 minutes, respectively. Further, the supernatant liquid of the mixture of the aqueous solution before the filtration and the water-absorbing resin may be used as the measurement sample.

· Step C)

In the step C), the amount of the water-absorbing resin in the measurement sample is calculated by the calibration curve created in the aforementioned step A) from the amount of the predetermined water-soluble cation measured in the aforementioned step B). Specifically, the water-soluble cation concentration or the value of the electrode potential measured in the step B is compared to the calibration curve created above, and the corresponding concentration of the water-absorbing resin derived from the measurement sample in the total ionic strength adjusting agent aqueous solution is calculated. Next, the amount of the water-absorbing resin in the measurement sample can be calculated from the calculated concentration of the water-absorbing resin derived from the measurement sample in the total ionic strength adjusting agent aqueous solution in consideration of the amount of the used total ionic strength adjusting agent.

In a case where the content of the water-absorbing resin in the hygienic material, the absorbent article, or the intermediate product in the manufacturing process thereof is measured, there is a case where the water-absorbing resin in the hygienic material, the absorbent article, or the intermediate product in the manufacturing process thereof absorbs moisture for a period from the manufacturing of the hygienic material, the absorbent article, or the intermediate product in the manufacturing process thereof to the measurement, and a difference between the moisture content of the water-absorbing resin in the reference sample used in the creation of the calibration curve and the moisture content of the water-absorbing resin in the measurement sample is generated. In this case, both the moisture content of the water-absorbing resin in the reference sample used in the creation of the calibration curve and the moisture content of the water-absorbing resin in the measurement sample are, for example, dried at 180°C for 3 hours and measured and then the difference therebetween is corrected, which is preferable in order to obtain a precise measurement result.

Further, in a case where an unknown content of the water-absorbing resin in a commercially available hygienic material, absorbent article, or intermediate product in a manufacturing process thereof is measured, the water-absorbing resin used in the creation of the calibration curve is extracted from the hygienic material, the absorbent article, or the intermediate product in the manufacturing process thereof and then measurement is performed. Therefore, it can be considered that the moisture contents are almost the same, and it is considered that correction by the moisture contents is not necessary to perform. However, there is no limitation that a person executing the present invention converts the moisture content to a specific moisture content.

Further, there is a case where a water-absorbing material or an absorber having an ion-dissociating group are separated into a plurality of layers, that is, an upper layer and a lower layer, or particularly, a product or a grade of the water-absorbing resin used in each layer is changed among the hygienic materials, absorbent articles, or intermediate products in a manufacturing process thereof which are marketed in recent years. Also in this case, when the water-absorbing resins in the absorbers of the respective layers are carefully separated to each other not to fly or be mixed, the present invention can be effectively applied to such a hygienic material, an absorbent article, or an intermediate product in the manufacturing process thereof by repeating the measuring method of the present invention in the respective layers.

(Correction of Error)

The above description is made on the assumption that the error which may be included in the measurement value of the amount of the predetermined water-soluble cation obtained in the measuring step M2) of the step B) is within a predetermined range. On the other hand, in a case where the error exceeds the predetermined range, the amount of the water-absorbing resin in the measurement sample cannot be always calculated with high precision on the basis of the measurement of the value of the water-soluble cation obtained in the measurement step M2) of the step C) even when the calibration curve with high precision can be created.

That is, as described above, the measurement method according to the present invention is a method for calculating the amount of the water-absorbing resin from the measurement value of the amount of the water-soluble cation in the water-absorbing resin included in the hygienic material, the absorbent article, or the intermediate product in the manufacturing process thereof, that is, the measurement sample, using the calibration curve created using the reference sample. Hence, for example, in a case where elements affecting the ion concentration are included in components other than the water-absorbing resin in the measurement sample, the error may be generated in the calculation value. In such a case, it may be necessary to verify the cause of the error and correct the calculation value according to the result.

As an example of the correction in a preferred embodiment, for example, there is exemplified an embodiment including a step for determining whether the error, which may be included in the measurement value obtained in the measuring step M2) of the step B), exceeds the predetermined range due to the "components other than the water-absorbing resin" in the measurement sample. Incidentally, as an example of a case where such an error is caused by the "components other than the water-absorbing resin" in the measurement sample, there is exemplified a case where the "components other than the water-absorbing resin" includes a predetermined water-soluble cation (for example, as an additive or the like) as the measurement target in the water-absorbing resin. Further, the same also applies to a case where the "components other than the water-absorbing resin" in the measurement sample has an adsorptive capacity with respect to the predetermined water-soluble cation as the measurement target in the water-absorbing resin.

A specific correction method is not particularly limited, but as a convenient method with high reliability, it is preferable to use a sample (blank sample) excluding the water-absorbing resin from the measurement sample used in the step B), measure the amount of the predetermined water-soluble cation derived from the blank sample, and determine whether the water-soluble cation is detected.

In a case where the water-soluble cation is detected from the blank sample or it is determined to have an adsorptive capacity with respect to the water-soluble cation, as the method for correcting an error, background processing is exemplified. Specifically, first, the amount of the predetermined water-soluble cation derived from the blank sample may be measured, and the measurement value of the amount of the water-soluble cation derived from the blank sample may be subtracted from the measurement value of the step C) on the basis of the obtained measurement result.

Alternatively, when the calibration curve is created, a reference sample further including, in addition to the known amount of the water-absorbing resin, a known amount of components other than the water-absorbing resin in the hygienic material, the absorbent article, or the intermediate product in the manufacturing process thereof is used. The calibration curve obtained in this way is made in consideration of an interaction between the water-absorbing resin and the components other than the water-absorbing resin in the hygienic material, the absorbent article, or the intermediate product in the manufacturing process thereof (for example, water-soluble cation adsorptive capacity). Therefore, it is possible to execute the measuring method according to the present invention with high precision even in a system in which the interaction exists by performing the step B) and the step C) using the calibration curve.

As described above, according to the method for measuring an amount of a water-absorbing resin according to the present invention, it is possible to measure the content of the water-absorbing resin in the hygienic material, the absorbent article, or the intermediate product in the manufacturing process thereof without using a strong acid, it is not necessary to perform titration using an acidic solution or a basic solution, and it is possible to perform a quantitative determination operation of the water-absorbing resin only by a safer reagent. Therefore, it is preferable to execute the measuring method according to the present invention by being incorporated in the method for manufacturing a hygienic material, an absorbent article, or an intermediate product in a manufacturing process thereof.

(Method for Manufacturing Hygienic Material, Absorbent Article, or Intermediate Product in Manufacturing Process thereof)

That is, according to another aspect of the present invention, there is also provided a method for manufacturing a hygienic material, an absorbent article, or an intermediate product in a manufacturing process thereof using the measurement method. Specifically, according to another preferred aspect of the present invention, there is provided a method for manufacturing a hygienic material or an absorbent article, the method including a step for producing a hygienic material, an absorbent article, or an intermediate product through feeding a predetermined amount of a water-absorbing resin, and a step for measuring an amount of the water-absorbing resin included in the hygienic material, the absorbent article, or the intermediate product in a manufacturing process thereof by the method for measuring an amount of a water-absorbing resin according to the aforementioned aspect of the present invention.

Herein, the specific configuration of the "hygienic material" or the "absorbent article" manufactured by the aforementioned manufacturing method is not particularly limited, and may include a conventionally known any product including a predetermined amount of the water-absorbing resin. Examples of the "hygienic material" include sanitary napkins, incontinence pads, and the like, in addition to paper diapers (for babies and for adults). Further, examples of the "absorbent article" include soil water retention agents, raising sheet, seed coating materials, dew condensation prevention sheets, drip absorbers, freshness-keeping materials, disposable body warmers, cooling bandanas, waste blood solidifying agents, surplus soil solidifying agent, water loss prevention waste liquid gelatinizers, absorption sandbags, portable toilets for disaster, fomentation materials, thickeners for cosmetics, waterproof materials for electric or electronic material communication cables, gasket packings, sustained preparations for fertilizer, pet sheet, cat sands, dressing materials for wound protection, building materials for dew condensation prevention, moisture-in-oil removers, and the like.

Of them, the hygienic material or the absorbent article preferably has a liquid permeable top sheet, a liquid impermeable back sheet joined to the top sheet, and an absorber (absorbent core) which exists between the top sheet and the back sheet and includes the water-absorbing resin, the hygienic material with such a configuration is more preferable, a paper diaper, a sanitary napkin, or an incontinence pad having such a configuration is particularly preferable, and a paper diaper having such a configuration is most preferable.

Incidentally, the hygienic material or the absorbent article may be a final product or an intermediate product. That is, the method for measuring a content of an absorbent resin according to the present invention can be applied to both a final product and an absorber (absorbent core) collected from an intermediate product in a manufacturing process thereof. Further, the measurement target is not limited to the absorber, and for example, may be members described below.

In a preferable embodiment of the manufacturing method according to the present embodiment, the absorber (absorbent core) further includes a fiber base material. In this case, the absorber may include a mixture in which the water-absorbing resin is dispersed in a fiber base material formed from natural fibers such as cellulose fiber and wood-crushed pulp or synthetic fibers such as cellulose acetate and rayon.

In another preferable embodiment of the manufacturing method according to the present invention, in the absorber (absorbent core), particles of the water-absorbing resin are attached to each other by a glue. In this case, the absorber (absorbent core may be in the form such as a water-absorbing resin sheet in which the water-absorbing resin is sandwiched by two sheets of upper and lower non-woven fabrics and fixed.

Further, the measuring method in the present invention can be executed also to the water-absorbing resin sheet before being cut per one sheet of the absorbent article. In this case, although not particularly limited, by cutting a certain area of the water-absorbing resin sheet from the water-absorbing resin sheet before being cut and quantitatively determining the amount of the water-absorbing resin in the water-absorbing resin sheet, the basis weight of the water-absorbing resin in the water-absorbing resin sheet can be obtained by calculation.

In still another preferable embodiment of the manufacturing method according to the present embodiment, the absorber (absorbent core) may be in the form such as a complex of fiber-water-absorbing resin obtained by immersing a monomer aqueous solution as a raw material of the water-absorbing resin in a fiber base material such as a non-woven fabric.

Incidentally, the absorber (absorbent core) may be an absorber (absorbent core) having a multi-layered structure in which the absorber (absorbent core) is separated into an upper layer and a lower layer.

The method for manufacturing an absorber (absorbent core) is not particularly limited, and conventionally known knowledge may be appropriately referred to, and for example, methods described in JP 10-507967 W (WO 96/13335 A), US 2003/130638 A, US 5102585, WO 00/33782 A, and the like are exemplified.

Further, also regarding the method for obtaining a hygienic material, an absorbent article, or an intermediate product in a manufacturing process thereof, since the hygienic material, the absorbent article, or intermediate product in the manufacturing process thereof as an application target of the present invention includes a predetermined amount of the water-absorbing resin, any manufacturing methods may be used as long as they are a method for obtaining a hygienic material, an absorbent article, or an intermediate product in a manufacturing process thereof through a step for feeding (quantitatively supplying) the predetermined amount of the water-absorbing resin. Examples of a method for obtaining a hygienic material or an absorbent article include methods described in JP 2010-246990 A, JP 2014-534820 W, JP 2017-509393 W, JP 2012-34715 A, JP 2009-284984 A, JP 2001-555620 W, JP 2013-99429 A, JP 6-181948 A, JP 2002-360630 A, JP 2001-309945 A, JP 2009-291475 A, and the like.

The method for manufacturing a hygienic material, an absorbent article, or an intermediate product in a manufacturing process thereof according to the aforementioned preferable embodiment of the present invention includes producing a hygienic material, an absorbent article, or an intermediate product in a manufacturing process thereof through a step for feeding (quantitatively supplying) the predetermined amount of the water-absorbing resin. The feeding (quantitatively supplying) of the predetermined amount of the water-absorbing resin can be executed using a conventionally known apparatus such as a screw feeder or a vibratory feeder. Herein, in producing of a hygienic material, an absorbent article, or an intermediate product in a manufacturing process thereof, regarding the step for obtaining an absorber (absorbent core) using a water-absorbing resin (water-absorbing agent) and a hydrophilic fiber (wood pulp), a case where a vibratory feeder is used as a means for feeding (quantitatively supplying) of the predetermined amount of the water-absorbing resin (water-absorbing agent) will be described as an example.

First, a water-absorbing resin (water-absorbing agent) is put in a hopper of a vibratory feeder, and the vibratory feeder is set such that the water-absorbing resin (water-absorbing agent) is intermittently dropped on a metallic mesh by switching on/off of the vibratory feeder in which the conveyance amount and the charging amount have been adjusted in advance. Meanwhile, another vibratory feeder is set such that the predetermined amount of the water-absorbing resin (water-absorbing agent) is dropped on a metallic mesh while the wood pulp is loosen from a time point before the water-absorbing resin (water-absorbing agent) is dropped to a time point when the dropping the predetermined amount of the water-absorbing resin (water-absorbing agent) ends. Next, the water-absorbing resin (water-absorbing agent) and the wood pulp are dropped from respective vibratory feeders on the metallic mesh having a predetermined size of a frame (for example, having an opening of 150 $\mu$m), and they are suctioned from the lower part of the metallic mesh, so that an absorber (absorbent core) can be manufactured. At this time, first, since the supplying of the wood pulp is started and a layer of the wood pulp is started to be formed on the metallic mesh, the vibratory feeder switch of supplying the water-absorbing resin (water-absorbing agent) is then turned on, the predetermined amount of the water-absorbing resin (water-absorbing agent) is dropped for a predetermined time, and thus the manufacturing of the absorber (absorbent core) can be completed.

As described above, in the measuring method according to the embodiment of the present invention, in the step B), the amount of the predetermined water-soluble cation, which includes a portion including the water-absorbing resin, in the measurement sample of the hygienic material, the absorbent article, or the intermediate product in the manufacturing process thereof is measured. At this time, after all, as described above, the measurement sample preferably includes "the whole amount of the water-absorbing resin," but a part of the water-absorbing resin may be included. Further, it is preferable to use a part including the whole amount of the water-absorbing resin as a target of quantitative determination as the measurement sample, and particularly, it is preferable to extract an absorber from the hygienic material, the absorbent article, or the intermediate product in the manufacturing process thereof and then use the absorber as the measurement sample.

Further, according to still another aspect of the present invention, in a method for continuously manufacturing a hygienic material, an absorbent article, or an intermediate product in a manufacturing process thereof, there is also provided a technique for feeding back a measurement result obtained by the method for measuring a water-absorbing resin according to an aspect of the present invention to the manufacturing step (changing a manufacturing condition of the hygienic material, the absorbent article, or the intermediate product in the manufacturing process thereof). Specifically, according to still another preferred aspect of the present invention, regarding a hygienic material, an absorbent article, or an intermediate product in a manufacturing process thereof continuously produced through a step for feeding a predetermined amount of the water-absorbing resin, there is provided a method for continuously manufacturing a hygienic material, an absorbent article, or an intermediate product in a manufacturing process thereof, the method including: a step for continuously producing a hygienic material, an absorbent article, or an intermediate product through feeding a predetermined amount of a water-absorbing resin, and a step for measuring an amount of the water-absorbing resin included in the hygienic material, the absorbent article, or the intermediate product in the manufacturing process thereof by the method for measuring an amount of a water-absorbing resin according to the aforementioned aspect of the present invention; and a step for changing a manufacturing condition of the hygienic material, the absorbent article, or the intermediate product in the manufacturing process thereof on the basis of the measurement result (hereinafter, also referred to as the "controlling step"), wherein the manufacturing condition is a setting value of feed amount of the water-absorbing resin in the step for feeding a predetermined amount of the water-absorbing resin, and wherein in a case where a calculation value of the amount of the water-absorbing resin in a measurement sample is below the lower limit of a predetermined range, the manufacturing condition is changed such that the amount of the water-absorbing resin supplied in the manufacturing step increases and in a case where the calculation value of the amount of the water-absorbing resin in the measurement sample is above the upper limit of the predetermined range, the manufacturing condition is changed such that the amount of the water-absorbing resin supplied in the manufacturing step decreases.

Incidentally, regarding the expression "continuously manufacturing" in the present specification, all steps not have to be continuous steps, and even in the case of including a batch step, practically, as long as those steps are interlocked and the manufacturing of a hygienic material, an absorbent article, or an intermediate product in a manufacturing process thereof is performed with a series of continuous flows, the manufacturing thereof is regarded as "continuously manufacturing."

The method for continuously manufacturing a hygienic material, an absorbent article, or an intermediate product in a manufacturing process thereof is suitably used in plant-scale continuous production in industrial production, and the method is preferably applied to giant scale continuous production that the production amount is preferably 10 sheet/min or more on one line, further preferably 100 sheet/min or more, and particularly preferably 200 sheet/min or more. Further, the upper limit of the production amount is not particularly limited, but, usually, is preferably 5000 sheet/min or less, more preferably 2000 sheet/min or less, and still more preferably 1000 sheet/min or less.

Herein, the hygienic material, the absorbent article, or the intermediate product in the manufacturing process thereof continuously produced through the step for feeding a predetermined amount of the water-absorbing resin is not particularly limited as long as it is a hygienic material, an absorbent article, or an intermediate product in a manufacturing process thereof obtained after the step for feeding a predetermined amount of the water-absorbing resin is performed, and specifically, the hygienic material, the absorbent article, or the intermediate product in the manufacturing process thereof means an absorber (absorbent core) or a precursor thereof obtained by feeding the predetermined amount of the water-absorbing resin from an apparatus feeding the predetermined amount of the water-absorbing resin, or an intermediate product or a final product thereof of a hygienic material or an absorbent article sampled from a manufacturing line after the manufacturing step of the absorber (absorbent core).

That is, the measurement target of the amount of the water-absorbing resin (the measurement sample in the step B) may be an absorber (absorbent core) or a precursor thereof obtained by feeding the predetermined amount of the water-absorbing resin from an apparatus in which the step for feeding a predetermined amount of the water-absorbing resin, or a hygienic material, an absorbent article, or an intermediate product in a manufacturing process thereof sampled from a manufacturing line after the manufacturing step of the absorber (absorbent core), or may be a product of a hygienic material or an absorbent article to be finally obtained. However, the measurement target is preferably a product of a hygienic material or an absorbent article to be finally obtained which exists in a final packaging step of a continuous manufacturing line and/or immediately before and/or near that step. Incidentally, in the case of measuring the content of the water-absorbing resin in an absorber (absorbent core) or a precursor thereof obtained by feeding the predetermined amount of the water-absorbing resin from an apparatus in which the step for feeding a predetermined amount of the water-absorbing resin or a hygienic material, an absorbent article, or an intermediate product in a manufacturing process thereof sampled from a manufacturing line after the manufacturing step of the absorber (absorbent core), the measurement place thereof is not particularly limited, and may be a place near the manufacturing step of the absorber (absorbent core) or a precursor thereof obtained by feeding the predetermined amount of the water-absorbing resin from an apparatus in which the step for feeding a predetermined amount of the water-absorbing resin or may be a place near the end of the manufacturing line (the packaging step after manufacturing the final hygienic material of absorbent article). To summarize this, the measurement sample in the step B) may be derived from a hygienic material, an absorbent article, or an intermediate product in the middle of a manufacturing line thereof or may be derived from a final product to be finally obtained through the manufacturing line of the hygienic material or the absorbent article, but is preferably derived from a final product.

The measuring of the amount of the water-absorbing resin in the measurement sample may be continuous measuring or may be performed by certain sampling for each predetermined production output and/or predetermined time. Incidentally, the expression "for each predetermined time" may mean, for example, for each lot or may be in the form of sampling inspection for each production output in a lot. More specifically, for example, in a case where final products are packaged for each predetermined amount, sampling inspection of corresponding product package is performed for each predetermined production output and/or predetermined time or sampling is performed with a line flow with respect to the lot.

In a preferable embodiment, the measuring of the content of the water-absorbing resin in the measurement sample is automatically and continuously performed. However, the measuring of the content of the water-absorbing resin in the measurement sample may be performed semi-automatically or manually.

Also in the method for continuously manufacturing a hygienic material, an absorbent article, or an intermediate product in a manufacturing process thereof, similarly to the method for manufacturing a hygienic material, an absorbent article, or an intermediate product in a manufacturing process thereof according to the aforementioned aspect, a step for measuring an amount of the water-absorbing resin in the hygienic material, the absorbent article, or the intermediate product in the manufacturing process thereof by the method for measuring an amount of a water-absorbing resin according to the aforementioned aspect of the present invention is performed. Further, the method for continuously manufacturing a hygienic material, an absorbent article, or an intermediate product in a manufacturing process thereof according to the present embodiment is characterized by including a step for changing a manufacturing condition of the hygienic material, the absorbent article, or the intermediate product in the manufacturing process thereof on the basis of the measurement result (controlling step). Such a controlling step is performed for the purpose of preventing occurrence of a subsequent out of spec product by detecting spec out when the amount of the water-absorbing resin in the hygienic material or the absorbent article continuously manufactured is out of spec, and performing feedback to change the manufacturing condition. Currently, since the manufacturing line of the hygienic material, the absorbent article, or the intermediate product in the manufacturing process thereof is continuously operated at a very high speed, in the case of using a conventional method for measuring a water-absorbing resin which takes a long time until a measurement result is obtained, even when the measurement result is fed back, a long time elapses during a period from sampling to changing of the manufacturing condition, and a problem arises in that a large amount of out of spec products occurs. On the other hand, according to the method for continuously manufacturing a hygienic material, an absorbent article, or an intermediate product in a manufacturing process thereof according to the present embodiment, it is possible to measure the amount of the water-absorbing resin in the hygienic material, the absorbent article, or the intermediate product in a manufacturing process thereof in an extremely short time. Therefore, it is possible to significantly shorten the period from sampling to changing of the manufacturing condition, and as a result, it is possible to suppress the occurrence amount of the out of spec product to the minimum and to stably manufacture a hygienic material, an absorbent article, or an intermediate product in a manufacturing process thereof. Hereinafter, the controlling step will be described in more detail.

When the amount of the water-absorbing resin in the hygienic material, the absorbent article, or the intermediate product in the manufacturing process thereof is measured by the method for measuring a content of a water-absorbing resin according to the aforementioned aspect of the present invention, the "amount of the water-absorbing resin in the measurement sample" is calculated as a result of undergoing the step C). In the controlling step, whether the calculation value is a value within a predetermined range of the content of the water-absorbing resin which has been set in advance is determined. Incidentally, the "predetermined range of the content of the water-absorbing resin" may be appropriately determined from a targeted content of the water-absorbing resin (target content). The method for determining a predetermined range of the content of the water-absorbing resin is not particularly limited, but for example, there is exemplified a method for determining a predetermined range of the content of the water-absorbing resin using, as an upper and lower limit, a value deviating to a predetermined value from the target content of the water-absorbing resin in a hygienic material, an absorbent article, or an intermediate product in a manufacturing process thereof which is desired to be finally obtained.

As a result of the determination, in a case where the value is a value within the predetermined range, subsequent particular controlling (changing of the manufacturing condition) is not performed. On the other hand, in a case where the calculation value of the "amount of the water-absorbing resin in the measurement sample" is not a value within the predetermined range which has been set in advance (is below the lower limit or above the upper limit of the predetermined range), subsequent controlling (changing of the manufacturing condition) is performed. Specifically, in a case where the calculation value of the "amount of the water-absorbing resin in the measurement sample" is below the lower limit of the predetermined range, the manufacturing condition of the hygienic material, the absorbent article, or the intermediate product in the manufacturing process thereof is changed such that the amount of the water-absorbing resin supplied in the manufacturing step of the absorber increases. On the other hand, in a case where the calculation value of the "amount of the water-absorbing resin in the measurement sample" is above the upper limit of the predetermined range, the manufacturing condition of the hygienic material, the absorbent article, or the intermediate product in the manufacturing process thereof is changed such that the amount of the water-absorbing resin supplied in the manufacturing step of the absorber decreases.

In such a controlling step, the manufacturing condition changed on the basis of the calculation value is a setting value of a feed amount of the water-absorbing resin in the step for feeding the predetermined amount of the water-absorbing resin. By changing the setting value, it is possible to directly increase or decrease the feed amount of the water-absorbing resin in the step for feeding the predetermined amount of the water-absorbing resin. Further, there is exemplified a method for adjusting the manufacturing line speed or the feed amount of other members such that the amount of the water-absorbing resin per one sheet (item) of the hygienic material, the absorbent article, or the intermediate product in the manufacturing process thereof is set to a setting value.

In the controlling step of the method for continuously manufacturing a hygienic material, an absorbent article, or an intermediate product in a manufacturing process thereof according to the present invention, it is possible to control a feed amount of the water-absorbing resin in the step for feeding a predetermined amount of the water-absorbing resin by changing at least one of those manufacturing conditions. Particularly, it is preferable to directly control the feed amount of the water-absorbing resin by changing a setting value of the feed amount of the water-absorbing resin in the step for feeding the predetermined amount of the water-absorbing resin. Also in the controlling step, it is preferable to automatically and continuously perform the changing of the manufacturing conditions based on the measurement result of the content of the water-absorbing resin in the measurement sample. However, also in the controlling step, the changing of the manufacturing conditions based on the measurement result of the content of the water-absorbing resin in the measurement sample may be performed semi-automatically or manually.

(Method for Measuring Water-Absorbing Resin)

The method for measuring an amount of a water-absorbing resin included in a hygienic material, an absorbent article, or an intermediate product in a manufacturing process thereof, wherein the water-absorbing resin includes a water-soluble cation derived from neutralization of an acid group of the water-absorbing resin, according to the present invention can also be executed by using the "water-absorbing resin which has a water-soluble cation in the form of an ion-dissociating group" itself as the measurement target. That is, the measuring method is not limited to the water-absorbing resin included in the hygienic material, the absorbent article, or the intermediate product in the manufacturing process thereof, but can be applied to a method for measuring an amount of a water-absorbing resin itself having a water-soluble cation in the form of an ion-dissociating group.

That is, the method for measuring a water-absorbing resin according to a preferred embodiment of the present invention is a method for measuring an amount of a water-absorbing resin which has a water-soluble cation in the form of an ion-dissociating group, the method including:

A) a step for measuring an amount of a predetermined water-soluble cation in a reference sample including a known amount of the water-absorbing resin and creating a calibration curve indicating a relation between the amount of the water-absorbing resin and the amount of the predetermined water-soluble cation in the reference sample on the basis of the obtained measurement result;
B) a step for measuring an amount of the predetermined water-soluble cation in a measurement sample, which includes a portion including the water-absorbing resin; and
C) a step for calculating an amount of the water-absorbing resin in the measurement sample by the calibration curve created in the step A) from the amount of the predetermined water-soluble cation measured in the step B),

the step A) and the step B) including measuring the amount of the predetermined water-soluble cation using an ion selective electrode method and including the following steps M1) and M2):

M1) a step for immersing a sample in an aqueous solution including a total ionic strength adjusting agent (pre-processing step); and
M2) a step for measuring the amount of the predetermined water-soluble cation in a pre-processed supernatant liquid obtained in the step M1).

Herein, preferably, the amount of the water-absorbing resin to be measured is a content of the water-absorbing resin in the hygienic material, the absorbent article, or the intermediate product in the manufacturing process thereof, and in this case, the measurement method is "the method for measuring a content of a water-absorbing resin, which has a water-soluble cation in the form of an ion-dissociating group, included in an absorbent article or an intermediate product in a manufacturing process thereof" of the present invention.

Herein, the water-absorbing resin in the present invention also includes an intermediate in a processing process of the water-absorbing resin, an amount of the water-absorbing resin in powder dust, and the amount of the water-absorbing resin in a water dispersion liquid or a water-swelling gel, and is not particularly limited. Other than, as a preferable condition of the measuring method, "the method for measuring a content of a water-absorbing resin, which has a water-soluble cation in the form of an ion-dissociating group, included in an absorbent article or an intermediate product in a manufacturing process thereof" of the present invention described above can be applied.

EXAMPLES

Hereinafter, examples of the method for measuring a content of a water-absorbing resin in an absorber according to the present invention will be described, but the present invention is not limited thereto.

[Example 1]

In this Example, as an ion meter equipped with an ion selective electrode, a compact sodium ion meter LAQUA twin B-722 manufactured by HORIBA, Ltd. was used and correction was performed in advance according to the manual. Incidentally, in this ion meter, the sodium ion concentration (ppm) in a sample solution can be measured.

(Creation of Calibration Curve by Known Amount of Water-Absorbing Resin)

In a 1000 mL plastic container, 500 mL of aqueous solution of 5% by weight potassium chloride (manufactured by Wako Pure Chemical Industries, Ltd., first class) set to 23°C was put, and 2.5 g of water-absorbing resin that is a known amount of partially neutralized polyacrylic acid sodium salt was put and swollen for 10 minutes while being stirred with a stirrer chip. Thereafter, stirring was stopped, and the mixture was left to stand still for 30 seconds and filtered to obtain a supernatant liquid. The obtained supernatant liquid was collected with a dropper and transferred to a sensor unit of the ion meter, and the sodium ion concentration in the supernatant liquid was measured.

The same operation as described above was performed while the weight of the water-absorbing resin was changed to 5.0 g or 7.5 g, the same operation was further performed without using the water-absorbing resin, and results of a sodium ion concentration (ppm) (longitudinal axis) of the supernatant liquid with respect to a water-absorbing resin weight (concentration: g/L) (horizontal axis) per 1 L of 5% by weight potassium chloride aqueous solution were plotted to create a graph. The results are shown in the following Table 1 and Fig. 1. Further, in Fig. 1, a calibration curve obtained by the least-square method (primary approximation formula) is shown along with a determination coefficient $R^2$ thereof. As shown in Fig. 1, the calibration curve could be created as a primary approximation formula in which the determination coefficient $R^2$ is extremely large of 0.9968 from four plots.

[Table 1]

| 5% by weight KCl aqueous solution (mL) | Water-absorbing resin weight (g) | Water-absorbing resin concentration | Na ion concentration (ppm) |
|---|---|---|---|
| | | (g/L) | |
| 500 | 0 | 0 | 180 |
| 500 | 2.5 | 5.0 | 950 |
| 500 | 5.0 | 10.0 | 1700 |
| 500 | 7.5 | 15.0 | 2300 |

(Quantitative Determination of Water-Absorbing Resin in Mixture of Known Amount of Water-Absorbing Resin and Pulp)

9.9 g of the water-absorbing resin used in the creation of the calibration curve and 10 g of wood-crushed pulp were dry mixed with a mixer to obtain a simulant absorber. The obtained simulant absorber was put in 1000 mL of 5% by weight potassium chloride aqueous solution collected in a 2000 mL polyjug and stirred for 10 minutes with a three-one motor. Incidentally, the liquid temperature of the used potassium chloride aqueous solution was 23°C. After stirring for 10 minutes, the mixture was left to stand still for 30 seconds and then filtered to obtain a supernatant liquid in the same manner as the creation of the calibration curve described above. The obtained supernatant liquid was collected with a dropper and added dropwise to the sensor unit of the compact sodium ion meter used in the creation of the calibration curve and the sodium ion concentration in the supernatant liquid was measured. As a result, the measured sodium ion concentration was 1600 ppm and the weight of the water-absorbing resin per 1 L of 5% by weight potassium chloride aqueous solution was calculated to be 9.7 g/L from the previously obtained calibration curve (Fig. 1). Herein, in this Example, the volume of the 5% by weight potassium chloride aqueous solution in which the simulant absorber is dispersed was 1000 mL, and from this, the weight of the water-absorbing resin in the simulant absorber was calculated to be 9.7 g.

In this way, an error with the weight of the water-absorbing resin actually put was 0.2 g, and the content of the water-absorbing resin in the simulant absorber could be measured with extremely high precision.

[Example 2]

The content of the water-absorbing resin in the simulant absorber was measured using a pH/ion water quality meter manufactured by Thermo Fisher Scientific Inc. instead of the compact sodium ion meter manufactured by HORIBA, Ltd. used in the Example 1. Incidentally, in the used pH/ion water quality meter, the main body is Orion Star A320 and the electrode is ORION 8611BNWP sodium composite electrode. Correction was performed in advance according to the manual.

Incidentally, in this ion meter, the sodium ion concentration in the sample solution was measured as an electrode potential, so that calculation was performed using Nernst's equation.

(Creation of Calibration Curve by Known Amount of Water-Absorbing Resin)

In a 1000 mL plastic container, 500 mL of aqueous solution of 5% by weight potassium chloride (manufactured by Wako Pure Chemical Industries, Ltd., first class) set to 23°C was put, and 2.5 g of water-absorbing resin that is a known amount of partially neutralized polyacrylic acid sodium salt was put and swollen for 10 minutes while being stirred with a stirrer chip. Thereafter, stirring was stopped, and the mixture was left to stand still for 30 seconds and filtered to obtain a supernatant liquid. The sodium ion electrode was immersed in the obtained supernatant liquid and then an electrode potential (mV) was measured.

The same operation as described above was performed while the weight of the water-absorbing resin was changed to 5.0 g or 7.5 g, and each electrode potential (mV) was measured. The measurement results are shown in the following Table 2. Further, a value of common logarithm (log[SAP conc.]) of a water-absorbing resin weight (concentration: SAP conc. [g/L]) per 1 L of 5% by weight potassium chloride aqueous solution was also shown in Table 2 along with the measurement results, and results of the electrode potential (mV) (longitudinal axis) with respect to a value of common logarithm (horizontal axis) of a water-absorbing resin concentration (g/L) were plotted to create a graph. The created graph is shown in Fig. 2. Further, in Fig. 2, a calibration curve (primary approximation formula) obtained by the least-square method is shown along with a determination coefficient $R^2$ thereof. As shown in Fig. 2, also by this method, the calibration curve could be created as a primary approximation formula in which the determination coefficient $R^2$ is extremely large of 1.

[Table 2]

| 5% by weight KCl aqueous solution (ml) | Water-absorbing resin weight (g) | Water-absorbing resin concentration (g/L) | log [SAP conc.] | Electrode potential E (mV) |
|---|---|---|---|---|
| 500 | 2.5 | 5.0 | 0.6990 | -36.7 |
| 500 | 5.0 | 10.0 | 1.0000 | -21.2 |
| 500 | 7.5 | 15.0 | 1.1761 | -12.0 |

(Quantitative Determination of Water-Absorbing Resin in Mixture of Known Amount of Water-Absorbing Resin and Pulp)

10.0 g of the water-absorbing resin used in the creation of the calibration curve and 10 g of wood-crushed pulp were dry mixed with a mixer to obtain a simulant absorber. The obtained simulant absorber was put in 1000 mL of 5% by weight potassium chloride aqueous solution collected in a 2000 mL polyjug and stirred for 10 minutes with a three-one motor. Incidentally, the liquid temperature of the used potassium chloride aqueous solution was 23°C. After stirring for 10 minutes, the mixture was left to stand still for 30 seconds and then filtered to obtain a supernatant liquid in the same manner as the creation of the calibration curve described above. The sodium ion electrode used in the creation of the calibration curve was immersed in the obtained supernatant liquid and then an electrode potential (mV) was measured. As a result, the potential measured at the sodium ion electrode was - 20.6 mV and the weight of the water-absorbing resin per 1 L of 5% by weight potassium chloride aqueous solution was calculated to be 10.2 g/L from the previously obtained calibration curve (Fig. 2). Herein, in this Example, the volume of the 5% by weight potassium chloride aqueous solution in which the simulant absorber is dispersed was 1000 mL, and from this, the weight of the water-absorbing resin in the simulant absorber was calculated to be 10.2 g.

In this way, an error with the weight of the water-absorbing resin actually put was 0.2 g, and the content of the water-absorbing resin in the simulant absorber could be measured with extremely high precision.

[Example 3]

In Example 2, a blind test was performed while a sample preparer and a sample measurer were separated. The sample preparer prepared the following three samples:

· Sample A obtained by mixing 9.7 g of the water-absorbing resin for creating a calibration curve and 10.3 g of the wood-crushed pulp used in the Example 2 in a similar manner to the Example 2;
· Sample B similarly obtained by mixing 10.1 g of the water-absorbing resin and 9.9 g of the wood-crushed pulp in a similar manner to the Example 2; and
· Sample C similarly obtained by mixing 10.3 g of the water-absorbing resin and 9.7 g of the wood-crushed pulp in a similar manner to the Example 2.

Further, only the contents that the weight of the water-absorbing resin in each sample was about 10 g were sent to the sample measurer and respective samples were delivered by hand. Thereafter, the sample measurer repeated the same operation as in the Example 2, the electrode potential of the supernatant liquid was measured, and the content of the water-absorbing resin in the sample was measured.

The measured electrode potential, the water-absorbing resin weight (concentration) per 1 L of 5% by weight potassium chloride aqueous solution estimated from the electrode potential by the calibration curve (Fig. 2), and the measurement value of the content of the water-absorbing resin calculated from the concentration are shown in the following Table 3.

[Table 3]

| Sample | Electrode potential E (mV) | log [SAP conc.] | SAP conc. (g/L) | Water-absorbing resin weight (measurement value) (g) | Water-absorbing resin weight (actual measurement value) (g) |
|---|---|---|---|---|---|
| A | -21.8 | 0.987 | 9.7 | 9.7 | 9.7 |
| B | -20.7 | 1.009 | 10.2 | 10.2 | 10.1 |
| C | -20.0 | 1.022 | 10.5 | 10.5 | 10.3 |

As understood from the results shown in Table 3, it was possible to measure the content of the water-absorbing resin with extremely high precision that an error between the actual charging amount and about 10 g of the water-absorbing resin weight is within $\pm 0.2$ g ($\pm 2\%$) even in the blind test.

[Example 4]

In this Example, in the same manner as the Example 1, a compact sodium ion meter LAQUA twin B-722 manufactured by HORIBA, Ltd. was used as an ion meter equipped with an ion selective electrode and correction was performed in advance according to the manual.

(Creation of Calibration Curve by Known Amount of Water-Absorbing Resin)

100 g or more in total of only the water-absorbing resin from a plurality of sheets of a commercially available diaper A was extracted.

In a 3000 mL polyjug, 1500 mL of aqueous solution of 5% by weight potassium chloride (manufactured by Wako Pure Chemical Industries, Ltd., first class) set to 23°C was put, and 2.0 g of water-absorbing resin that is extracted from a known amount of commercially available diaper A was put and swollen for 10 minutes while being stirred with a three-one motor at 250 rpm. Thereafter, stirring was stopped, and the mixture was left to stand still for 30 seconds and filtered to obtain a supernatant liquid. The obtained supernatant liquid was collected with a dropper and transferred to a sensor unit of the ion meter, and the sodium ion concentration in the supernatant liquid was measured.

The same operation as described above was performed while the weight of the water-absorbing resin was changed to 4.0 g, 8.0 g, 12.0 g, and 16.0 g, the same operation was further performed without using the water-absorbing resin, and results of a sodium ion concentration (ppm) (longitudinal axis) of the supernatant liquid with respect to a water-absorbing resin weight (concentration: g/L) (horizontal axis) per 1 L of 5% by weight potassium chloride aqueous solution were plotted to create a graph. The results are shown in the following Table 4 and Fig. 3. Further, in Fig. 3, a calibration curve obtained by the least-square method (primary approximation formula) is shown along with a determination coefficient $R^2$ thereof. As shown in Fig. 3, the calibration curve could be created as a primary approximation formula in which the determination coefficient $R^2$ is extremely large of 0.9986 from six plots.

[Table 4]

| 5% by weight KCl aqueous solution (mL) | Water-absorbing resin weight (g) | Water-absorbing resin concentration (g/L) | Na ion concentration (ppm) |
|---|---|---|---|
| 1500 | 0 | 0 | 160 |
| 1500 | 2.0 | 1.3 | 350 |
| 1500 | 4.0 | 2.7 | 540 |

(continued)

| 5% by weight KCl aqueous solution (mL) | Water-absorbing resin weight (g) | Water-absorbing resin concentration (g/L) | Na ion concentration (ppm) |
|---|---|---|---|
| 1500 | 8.0 | 5.3 | 900 |
| 1500 | 12.0 | 8.0 | 1300 |
| 1500 | 16.0 | 10.7 | 1600 |

(Quantitative Determination of Water-Absorbing Resin in Commercially Available Diaper)

After portions other than the absorber (the side gather, the waist part, the tape, and the like) were removed from the commercially available diaper A, the diaper was cut into about 5 × 5 cm square such that the water-absorbing resin was not spilt. The cut diaper was put in 1500 mL of 5% by weight potassium chloride aqueous solution collected in a 3000 mL polyjug and stirred for 10 minutes with a three-one motor at 250 rpm. Incidentally, the liquid temperature of the used potassium chloride aqueous solution was 23°C. After stirring for 10 minutes, in the same manner as the creation of the calibration curve described above, the mixture was left to stand still for 30 seconds and then filtered to obtain a supernatant liquid. The obtained supernatant liquid was collected with a dropper and added dropwise to the sensor unit of the compact sodium ion meter used in the creation of the calibration curve and the sodium ion concentration in the supernatant liquid was measured. As a result, the measured sodium ion concentration was 960 ppm and the weight of the water-absorbing resin per 1 L of 5% by weight potassium chloride aqueous solution was calculated to be 5.8 g/L from the previously obtained calibration curve (Fig. 3). Herein, in this Example, the volume of the 5% by weight potassium chloride aqueous solution in which the simulant absorber extracted from the commercially available diaper A is dispersed was 1500 mL, and from this, the weight of the water-absorbing resin in the simulant absorber was calculated to be 8.7 g.

Incidentally, the present application is based on Japanese Patent Application No. 2016-194387 filed on September 30, 2016.

## Claims

1. A method for measuring an amount of a water-absorbing resin included in a hygienic material, an absorbent article, or an intermediate product in a manufacturing process thereof, wherein the water-absorbing resin includes a water-soluble cation derived from neutralization of an acid group of the water-absorbing resin, the method comprising:

   A) a step for measuring an amount of a predetermined water-soluble cation in a reference sample including a known amount of the water-absorbing resin and creating a calibration curve indicating a relation between the amount of the water-absorbing resin and the amount of the predetermined water-soluble cation in the reference sample on the basis of the obtained measurement result;
   B) a step for measuring an amount of the predetermined water-soluble cation in a measurement sample, which includes a portion including the water-absorbing resin, of the hygienic material, the absorbent article, or the intermediate product in the manufacturing process thereof; and
   C) a step for calculating an amount of the water-absorbing resin in the measurement sample by the calibration curve created in the step A) from the amount of the predetermined water-soluble cation measured in the step B), the step A) and the step B) including measuring the amount of the predetermined water-soluble cation using an ion selective electrode method and including the following steps M1) and M2):

   M1) a step for immersing a sample in an aqueous solution including a total ionic strength adjusting agent (pre-processing step); and
   M2) a step for measuring the amount of the predetermined water-soluble cation in a pre-processed supernatant liquid obtained in the step M1),

   wherein the water-soluble cation is selected from a sodium ion, a potassium ion and an ammonium ion, and wherein the total ionic strength adjusting agent is selected from a potassium salt, a calcium salt, a magnesium salt and an ammonium salt when the water-soluble cation is a sodium ion; the total ionic strength adjusting agent is selected from a sodium salt, a calcium salt, a magnesium salt and an ammonium salt when the water-soluble cation is a potassium ion; and the total ionic strength adjusting agent is selected from a sodium salt, a potassium salt, a calcium salt and a magnesium salt when the water-soluble cation is an ammonium ion.

2. The measuring method according to claim 1, wherein the step B) further includes, before the step M1) in the step B), a step B1) for collecting the portion including the water-absorbing resin from the hygienic material, the absorbent article, or the intermediate product in the manufacturing process thereof and the portion including the water-absorbing resin collected in the step B1) is used as the measurement sample.

3. The measuring method according to claim 1 or 2, wherein a conversion formula of the calibration curve is obtained by the least-square method in the step A), wherein the creation of the calibration curve is repeatedly performed until a determination coefficient $R^2$ in an approximation formula of the created calibration curve becomes a predetermined threshold or more.

4. The measuring method according to any one of claims 1 to 3, wherein, when a water-absorbing resin before being incorporated into the hygienic material, the absorbent article, or the intermediate product in the manufacturing process thereof is available, the water-absorbing resin is used as the reference sample.

5. The measuring method according to any one of claims 1 to 3, wherein, when a water-absorbing resin before being incorporated into the hygienic material, the absorbent article, or the intermediate product in the manufacturing process thereof is not available, the step A) further includes, before the step M1) in the step A), a step A1) for collecting the water-absorbing resin from the hygienic material or the absorbent article and the water-absorbing resin collected in the step A1) is used as the reference sample.

6. The measuring method according to claim 5, wherein, when the water-absorbing resin is collected from the hygienic material, the absorbent article, or the intermediate product in the manufacturing process thereof in the step A1), the step A) further includes a step A2) for measuring a weight of the water-absorbing resin collected from the hygienic material, the absorbent article, or the intermediate product in the manufacturing process thereof and determining a range of the amount of the water-absorbing resin in the calibration curve to be created on the basis of the obtained measurement result.

7. The measuring method according to any one of claims 1 to 6, further comprising a step L) for determining the type of the water-soluble cation, when the type of the water-soluble cation of the water-absorbing resin is not known, before the step M1) of the step A).

8. The measuring method according to any one of claims 1 to 7, wherein the water-absorbing resin is a polyacrylic acid salt.

9. The measuring method according to any one of claims 1 to 8, wherein the water-soluble cation of the water-absorbing resin is a sodium ion.

10. The measuring method according to any one of claims 1 to 9, further comprising a step N) for selecting a total ionic strength adjusting agent to be used depending on the type of the water-soluble cation to be measured by the ion selective electrode method.

11. The measuring method according to any one of claims 1 to 10, wherein the total ionic strength adjusting agent is potassium chloride.

12. The measuring method according to any one of claims 1 to 11, wherein stirring is performed for at least one second or longer when the reference sample or the measurement sample is pre-processed with the aqueous solution including a total ionic strength adjusting agent.

13. A method for manufacturing a hygienic material or an absorbent article, the method comprising a step for producing a hygienic material, an absorbent article, or an intermediate product through feeding a predetermined amount of a water-absorbing resin, and a step for measuring an amount of the water-absorbing resin included in the hygienic material, the absorbent article, or the intermediate product in a manufacturing process thereof by the measuring method according to any one of claims 1 to 12.

14. A method for continuously manufacturing a hygienic material or an absorbent article, the method comprising:

a step for continuously producing a hygienic material, an absorbent article, or an intermediate product through feeding a predetermined amount of a water-absorbing resin, and a step for measuring an amount of the water-absorbing resin included in the hygienic material, the absorbent article, or the intermediate product in a manufacturing process thereof by the measuring method according to any one of claims 1 to 12; and

a step for changing a manufacturing condition of the hygienic material, the absorbent article, or the intermediate product in the manufacturing process thereof on the basis of the measurement result,

wherein the manufacturing condition is a setting value of feed amount of the water-absorbing resin in the step for feeding a predetermined amount of the water-absorbing resin, and

wherein in a case where a calculation value of the amount of the water-absorbing resin in a measurement sample is below the lower limit of a predetermined range, the manufacturing condition is changed such that the amount of the water-absorbing resin supplied in the manufacturing step increases and in a case where the calculation value of the amount of the water-absorbing resin in the measurement sample is above the upper limit of the predetermined range, the manufacturing condition is changed such that the amount of the water-absorbing resin supplied in the manufacturing step decreases.

15. The manufacturing method according to claim 13 or 14, wherein the measurement sample in the step B) of the measuring method is derived from a final product of the hygienic material or the absorbent article, or the intermediate product in the manufacturing process of the hygienic material or the absorbent article.

16. The manufacturing method according to any one of claims 13 to 15, wherein the hygienic material or the absorbent article has

a liquid permeable top sheet,
a liquid impermeable back sheet joined to the top sheet, and
an absorber existing between the top sheet and the back sheet and including the water-absorbing resin.

17. The manufacturing method according to claim 16, wherein the absorber further includes a fiber base material.

18. The manufacturing method according to claim 16, wherein particles of the water-absorbing resin are attached to each other by a glue in the absorber.

19. The manufacturing method according to claim 16 or 17, wherein the absorber included in the hygienic material or the absorbent article is a water-absorbing resin sheet, and
the water-absorbing resin sheet has a multi-layered structure in which the water-absorbing resin is sandwiched by non-woven fabrics.

**Patentansprüche**

1. Verfahren zur Messung einer Menge eines wasserabsorbierenden Harzes, das in ein hygienisches Material, einen saugfähigen Artikel oder ein Zwischenprodukt in einem Herstellungsverfahren dafür eingeschlossen ist, bei dem das wasserabsorbierende Harz ein wasserlösliches Kation einschließt, das von der Neutralisation einer Säuregruppe des wasserabsorbierenden Harzes stammt, wobei das Verfahren die folgenden Schritte umfasst:

A) einen Schritt zur Messung einer Menge eines vorgegebenen wasserlöslichen Kations in einer Referenzprobe, die eine bekannte Menge des wasserabsorbierenden Harzes einschließt, und Erstellung einer Kalibrierungskurve, die einen Zusammenhang zwischen der Menge des wasserabsorbierenden Harzes und der Menge des vorgegebenen wasserlöslichen Kations in der Referenzprobe auf Basis des erhaltenen Messergebnisses angibt;
B) einen Schritt zur Messung einer Menge des vorgegebenen wasserlöslichen Kations in einer Messprobe, die einen Teil, der das wasserlösliche Harz des hygienischen Materials, des saugfähigen Artikels oder des Zwischenprodukts in dem Herstellungsverfahren dafür einschließt, einschließt; und
C) einen Schritt zur Berechnung einer Menge des wasserabsorbierenden Harzes in der Messprobe durch die in Schritt A) erstellte Kalibrierungskurve aus der Menge des vorgegebenen wasserlöslichen Kations, die in Schritt B) gemessen wurde,
wobei der Schritt A) und der Schritt B) die Messung der Menge des vorgegebenen wasserlöslichen Kations unter Verwendung einer Methode mit ionenselektiver Elektrode einschließen und die folgenden Schritte M1) und M2) einschließen:

M1) einen Schritt zum Eintauchen einer Probe in eine wässrige Lösung, die ein Mittel zur Anpassung der Gesamtionenstärke einschließt (Vorverarbeitungsschritt); und

M2) einen Schritt zur Messung der Menge des vorgegebenen wasserlöslichen Kations in einer in Schritt M1) erhaltenen vorverarbeiteten Überstandflüssigkeit,

bei dem das wasserlösliche Kation ausgewählt aus einem Natriumion, einem Kaliumion und einem Ammoniumion und

bei dem das Mittel zur Anpassung der Gesamtionenstärke ausgewählt ist aus einem Kaliumsalz, einem Calciumsalz, einem Magnesiumsalz und einem Ammoniumsalz, wenn das wasserlösliche Kation ein Natriumion ist; das Mittel zur Anpassung der Gesamtionenstärke ausgewählt ist aus einem Natriumsalz, einem Calciumsalz, einem Magnesiumsalz und einem Ammoniumsalz, wenn das wasserlösliche Kation ein Kaliumion ist; und das Mittel zur Anpassung der Gesamtionenstärke ausgewählt ist aus einem Natriumsalz, einem Kaliumsalz, einem Calciumsalz und einem Magnesiumsalz, wenn das wasserlösliche Kation ein Ammoniumion ist.

2. Messverfahren gemäß Anspruch 1, bei dem Schritt B) ferner vor dem Schritt M1) in dem Schritt B) einen Schritt B1) einschließt zum Sammeln des Teils, der das wasserabsorbierende Harz aus dem hygienischen Material, dem saugfähigen Artikel oder dem Zwischenprodukt in dem Herstellungsverfahren dafür, einschließt, und der Teil, der das in Schritt B1) gesammelte wasserabsorbierende Harz einschließt, als die Messprobe verwendet wird.

3. Messverfahren gemäß Anspruch 1 oder 2, bei dem eine Umwandlungsformel der Kalibrierungskurve erhalten wird durch die Methode der kleinsten Quadrate in Schritt A), wobei die Erstellung der Kalibrierungskurve wiederholt durchgeführt wird, bis ein Bestimmungskoeffizient $R^2$ in einer Näherungsformel der erstellten Kalibrierungskurve ein vorgegebener Schwellenwert oder mehr wird.

4. Messverfahren gemäß mindestens einem der Ansprüche 1 bis 3, bei dem, wenn ein wasserabsorbierendes Harz, bevor es in das hygienische Material, den saugfähigen Artikel oder das Zwischenprodukt in dem Herstellungsverfahren dafür eingearbeitet wird, zur Verfügung steht, das wasserabsorbierende Harz als die Referenzprobe verwendet wird.

5. Messverfahren gemäß mindestens einem der Ansprüche 1 bis 3, bei dem, wenn ein wasserabsorbierendes Harz, bevor es in das hygienische Material, den saugfähigen Artikel oder das Zwischenprodukt in dem Herstellungsverfahren dafür eingearbeitet wird, nicht zur Verfügung steht, der Schritt A) ferner vor dem Schritt M1) in Schritt A) einen Schritt A1) einschließt zum Sammeln des wasserabsorbierenden Harzes aus dem hygienischen Material oder dem saugfähigen Artikel und das in dem Schritt A1) gesammelte wasserabsorbierende Harz als die Referenzprobe verwendet wird.

6. Messverfahren gemäß Anspruch 5, bei dem, wenn das wasserabsorbierende Harz aus dem hygienischen Material, dem saugfähigen Artikel oder dem Zwischenprodukt in dem Herstellungsverfahren dafür in Schritt A1 gesammelt wird, der Schritt A) ferner einen Schritt A2) zur Messung eines Gewichts des wasserabsorbierenden Harzes, das aus dem hygienischen Material, dem saugfähigen Artikel oder dem Zwischenprodukt in dem Herstellungsverfahren dafür gesammelt wurde, und Bestimmung eines Bereichs der Menge des wasserabsorbierenden Harzes in der Kalibrierungskurve, die auf Basis des erhaltenen Messergebnisses erstellt werden soll, einschließt.

7. Messverfahren gemäß mindestens einem der Ansprüche 1 bis 6, das ferner, wenn die Art des wasserlöslichen Kations des wasserabsorbierenden Harzes nicht bekannt ist, einen Schritt L) zur Bestimmung der Art des wasserlöslichen Kations vor dem Schritt M1) des Schritts A) umfasst.

8. Messverfahren gemäß mindestens einem der Ansprüche 1 bis 7, bei dem das wasserabsorbierende Harz ein Polyacrylsäuresalz ist.

9. Messverfahren gemäß mindestens einem der Ansprüche 1 bis 8, bei dem das wasserlösliche Kation des wasserabsorbierenden Harzes ein Natriumion ist.

10. Messverfahren gemäß mindestens einem der Ansprüche 1 bis 9, das ferner einen Schritt N) zur Auswahl eines Mittels zur Anpassung der Gesamtionenstärke, das abhängig von der Art des wasserlöslichen Kations, das durch die Methode mit ionenselektiver Elektrode gemessen werden soll, umfasst.

11. Messverfahren gemäß mindestens einem der Ansprüche 1 bis 10, bei dem das Mittel zur Anpassung der Gesamtionenstärke Kaliumchlorid ist.

12. Messverfahren gemäß mindestens einem der Ansprüche 1 bis 11, bei dem Rühren für mindestens eine Sekunde oder länger durchgeführt wird, wenn die Referenzprobe oder die Messprobe mit der wässrigen Lösung, die ein Mittel zur Anpassung der Gesamtionenstärke einschließt, vorverarbeitet wird.

13. Verfahren zur Herstellung eines hygienischen Materials oder eines saugfähigen Artikels, wobei das Verfahren einen Schritt zur Herstellung eines hygienischen Materials, eines saugfähigen Artikels oder eines Zwischenprodukts durch Zufuhr einer vorgegebenen Menge eines wasserabsorbierenden Harzes, und einen Schritt zur Messung einer Menge des wasserabsorbierenden Harzes, das in das hygienische Material, den saugfähigen Artikel oder das Zwischenprodukt in einem Herstellungsverfahren dafür eingeschlossen ist, mit dem Messverfahren gemäß mindestens einem der Ansprüche 1 bis 12 umfasst.

14. Verfahren zur kontinuierlichen Herstellung eines hygienischen Materials oder eines saugfähigen Artikels, wobei das Verfahren die folgenden Schritte umfasst:

einen Schritt zur kontinuierlichen Herstellung eines hygienischen Materials, eines saugfähigen Artikels oder eines Zwischenprodukts durch Zufuhr einer vorgegebenen Menge eines wasserabsorbierenden Harzes und einen Schritt zur Messung einer Menge des wasserabsorbierenden Harzes, das in das hygienische Material, den saugfähigen Artikel oder das Zwischenprodukt in einem Herstellungsverfahren dafür mit dem Messverfahren gemäß mindestens einem der Ansprüche 1 bis 12; und
einen Schritt zur Änderung einer Herstellungsbedingung für das hygienische Material, den saugfähigen Artikel oder das Zwischenprodukt in dem Herstellungsverfahren dafür auf Basis des Messergebnisses,
bei dem die Herstellungsbedingung ein Einstellungswert der Zufuhrmenge des wasserabsorbierenden Harzes in dem Schritt zur Zufuhr einer vorgegebenen Menge des wasserabsorbierenden Harzes ist und
bei dem in einem Fall, dass ein Berechnungswert der Menge des wasserabsorbierenden Harzes in einer Messprobe unterhalb der Untergrenze eines vorgegebenen Bereichs liegt, die Herstellungsbedingung so verändert wird, dass die Menge des wasserabsorbierenden Harzes, die in dem Herstellungsschritt zugeführt wird, zunimmt, und in einem Fall, dass der Berechnungswert der Menge des wasserabsorbierenden Harzes in der Messprobe oberhalb der Obergrenze des vorgegebenen Bereichs liegt, die Messbedingung so geändert wird, dass die Menge des wasserabsorbierenden Harzes, die in dem Herstellungsschritt zugeführt wird, abnimmt.

15. Herstellungsverfahren gemäß Anspruch 13 oder 14, bei dem die Messprobe in Schritt B) der Messmethode abgeleitet ist aus einem Endprodukt des hygienischen Materials oder des saugfähigen Artikels oder dem Zwischenprodukt in dem Herstellungsverfahren des hygienischen Materials oder des saugfähigen Artikels.

16. Herstellungsverfahren gemäß mindestens einem der Ansprüche 13 bis 15, bei dem das hygienische Material oder der saugfähigen Artikel

ein flüssigkeitsdurchlässiges Top-Sheet,
ein flüssigkeitsundurchlässiges Back-Sheet, das mit dem Top-Sheet verbunden ist, und
einen Absorber, der zwischen dem Top-Sheet und dem Back-Sheet vorliegt und das wasserabsorbierende Harz einschließt,
aufweist.

17. Herstellungsverfahren gemäß Anspruch 16, bei dem der Absorber ferner ein Material auf Faserbasis einschließt.

18. Herstellungsverfahren gemäß Anspruch 16, bei dem Partikel des wasserabsorbierenden Harzes in dem Absorber mit einem Klebstoff miteinander verbunden sind.

19. Herstellungsverfahren gemäß Anspruch 16 oder 17, bei dem der Absorber, der in das hygienische Material oder den saugfähigen Artikel eingeschlossen ist, ein wasserabsorbierendes Harz-Sheet ist und das wasserabsorbierende Harz-Sheet eine Mehrschichtstruktur aufweist, in der das wasserabsorbierende Harz durch Vliesstoffe eingeklemmt ist.

**EP 3 521 817 B1**

**Revendications**

1. Procédé de mesure d'une quantité d'une résine absorbant l'eau incluse dans un matériau hygiénique, un article absorbant ou un produit intermédiaire dans un procédé de fabrication de celui-ci, dans lequel la résine absorbant l'eau inclut un cation hydrosoluble dérivé de la neutralisation d'un groupe acide de la résine absorbant l'eau, le procédé comprenant :

A) une étape de mesure d'une quantité d'un cation hydrosoluble prédéterminé dans un échantillon de référence incluant une quantité connue de la résine absorbant l'eau et de création d'une courbe d'étalonnage indiquant une relation entre la quantité de la résine absorbant l'eau et la quantité du cation hydrosoluble prédéterminé dans l'échantillon de référence sur la base du résultat de mesure obtenu ;
B) une étape de mesure d'une quantité du cation hydrosoluble prédéterminé dans un échantillon de mesure, qui inclut une partie incluant la résine absorbant l'eau, du matériau hygiénique, de l'article absorbant ou du produit intermédiaire dans le procédé de fabrication de celui-ci ; et
C) une étape de calcul d'une quantité de la résine absorbant l'eau dans l'échantillon de mesure par la courbe d'étalonnage créée dans l'étape A) à partir de la quantité du cation hydrosoluble prédéterminé mesurée dans l'étape B),
l'étape A) et l'étape B) incluant le fait de mesurer la quantité du cation hydrosoluble prédéterminé en utilisant un procédé par électrode sélective d'ions et incluant les étapes M1) et M2) suivantes :

M1) une étape d'immersion d'un échantillon dans une solution aqueuse incluant un agent d'ajustement de la force ionique totale (étape de prétraitement) ; et
M2) une étape de mesure de la quantité du cation hydrosoluble prédéterminé dans un liquide surnageant prétraité obtenu dans l'étape M1),

dans lequel le cation hydrosoluble est sélectionné parmi un ion de sodium, un ion de potassium et un ion ammonium, et
dans lequel l'agent d'ajustement de la force ionique totale est sélectionné parmi un sel de potassium, un sel de calcium, un sel de magnésium et un sel d'ammonium lorsque le cation hydrosoluble est un ion de sodium ; l'agent d'ajustement de la force ionique totale est sélectionné parmi un sel de sodium, un sel de calcium, un sel de magnésium et un sel d'ammonium lorsque le cation hydrosoluble est un ion de potassium ; et l'agent d'ajustement de la force ionique totale est sélectionné parmi un sel de sodium, un sel de potassium, un sel de calcium et un sel de magnésium lorsque le cation hydrosoluble est un ion ammonium.

2. Procédé de mesure selon la revendication 1, dans lequel l'étape B) inclut en outre, avant l'étape M1) de l'étape B), une étape B1) de collecte de la partie incluant la résine absorbant l'eau provenant du matériau hygiénique, de l'article absorbant ou du produit intermédiaire dans le procédé de fabrication de celui-ci et la partie incluant la résine absorbant l'eau collectée dans l'étape B1) est utilisée comme échantillon de mesure.

3. Procédé de mesure selon la revendication 1 ou la revendication 2, dans lequel une formule de conversion de la courbe d'étalonnage est obtenue par la méthode des moindres carrés dans l'étape A), dans lequel la création de la courbe d'étalonnage est mise en oeuvre à plusieurs reprises jusqu'à ce qu'un coefficient de détermination $R^2$ dans une formule d'approximation de la courbe d'étalonnage créée devienne un seuil prédéterminé ou plus.

4. Procédé de mesure selon l'une quelconque des revendications 1 à 3, dans lequel, lorsqu'une résine absorbant l'eau avant d'être incorporée dans le matériau hygiénique, l'article absorbant ou le produit intermédiaire dans le procédé de fabrication de celui-ci est disponible, la résine absorbant l'eau est utilisée comme échantillon de référence.

5. Procédé de mesure selon l'une quelconque des revendications 1 à 3, dans lequel, lorsqu'une résine absorbant l'eau avant d'être incorporée dans le matériau hygiénique, l'article absorbant ou le produit intermédiaire dans le procédé de fabrication de celui-ci n'est pas disponible, l'étape A) inclut en outre, avant l'étape M1) de l'étape A), une étape A1) de collecte de la résine absorbant l'eau à partir du matériau hygiénique ou de l'article absorbant et la résine absorbant l'eau collectée dans l'étape A1) est utilisée comme échantillon de référence.

6. Procédé de mesure selon la revendication 5, dans lequel, lorsque la résine absorbant l'eau est collectée à partir du matériau hygiénique, de l'article absorbant ou du produit intermédiaire dans le procédé de fabrication de celui-ci dans l'étape A1), l'étape A) inclut en outre une étape A2) de mesure d'un poids de la résine absorbant l'eau collectée à partir du matériau hygiénique, de l'article absorbant ou du produit intermédiaire dans le procédé de fabrication de celui-ci et de détermination d'une plage de la quantité de la résine absorbant l'eau dans la courbe d'étalonnage à créer sur la base du résultat de mesure obtenu.

7. Procédé de mesure selon l'une quelconque des revendications 1 à 6, comprenant en outre une étape L) de détermination du type du cation hydrosoluble, lorsque le type du cation hydrosoluble de la résine absorbant l'eau n'est pas connu, avant l'étape M1) de l'étape A).

8. Procédé de mesure selon l'une quelconque des revendications 1 à 7, dans lequel la résine absorbant l'eau est un sel de polyacide acrylique.

9. Procédé de mesure selon l'une quelconque des revendications 1 à 8, dans lequel le cation hydrosoluble de la résine absorbant l'eau est un ion de sodium.

10. Procédé de mesure selon l'une quelconque des revendications 1 à 9, comprenant en outre une étape N) de sélection d'un agent d'ajustement de la force ionique totale à utiliser en fonction du type de cation hydrosoluble à mesurer par le procédé par électrode sélective d'ions.

11. Procédé de mesure selon l'une quelconque des revendications 1 à 10, dans lequel l'agent d'ajustement de la force ionique totale est le chlorure de potassium.

12. Procédé de mesure selon l'une quelconque des revendications 1 à 11, dans lequel une agitation est mise en oeuvre pendant au moins une seconde ou plus lorsque l'échantillon de référence ou l'échantillon de mesure est prétraité avec la solution aqueuse incluant un agent d'ajustement de la force ionique totale.

13. Procédé de fabrication d'un matériau hygiénique ou d'un article absorbant, le procédé comprenant une étape de production d'un matériau hygiénique, d'un article absorbant ou d'un produit intermédiaire par l'introduction d'une quantité prédéterminée d'une résine absorbant l'eau, et une étape de mesure d'une quantité de la résine absorbant l'eau incluse dans le matériau hygiénique, l'article absorbant ou le produit intermédiaire dans un procédé de fabrication de celui-ci par le procédé de mesure selon l'une quelconque des revendications 1 à 12.

14. Procédé de fabrication en continu d'un matériau hygiénique ou d'un article absorbant, le procédé comprenant :

une étape de production en continu d'un matériau hygiénique, d'un article absorbant ou d'un produit intermédiaire par l'introduction d'une quantité prédéterminée d'une résine absorbant l'eau, et une étape de mesure d'une quantité de la résine absorbant l'eau incluse dans le matériau hygiénique, l'article absorbant ou le produit intermédiaire dans un procédé de fabrication de celui-ci par le procédé de mesure selon l'une quelconque des revendications 1 à 12 ; et
une étape de modification d'une condition de fabrication du matériau hygiénique, de l'article absorbant ou du produit intermédiaire dans le procédé de fabrication de celui-ci sur la base du résultat de mesure,
dans lequel la condition de fabrication est une valeur d'ajustement de la quantité d'alimentation de la résine absorbant l'eau dans l'étape d'introduction d'une quantité prédéterminée de la résine absorbant l'eau, et
dans lequel dans un cas où une valeur de calcul de la quantité de la résine absorbant l'eau dans un échantillon de mesure est inférieure à la limite inférieure d'une plage prédéterminée, la condition de fabrication est modifiée de telle sorte que la quantité de la résine absorbant l'eau fournie dans l'étape de fabrication augmente et dans un cas où la valeur de calcul de la quantité de la résine absorbant l'eau dans l'échantillon de mesure est supérieure à la limite supérieure de la plage prédéterminée, la condition de fabrication est modifiée de telle sorte que la quantité de la résine absorbant l'eau fournie dans l'étape de fabrication diminue.

15. Procédé de fabrication selon la revendication 13 ou la revendication 14, dans lequel l'échantillon de mesure dans l'étape B) du procédé de mesure est dérivé d'un produit final du matériau hygiénique ou de l'article absorbant, ou du produit intermédiaire dans le procédé de fabrication du matériau hygiénique ou de l'article absorbant.

16. Procédé de fabrication selon l'une quelconque des revendications 13 à 15, dans lequel le matériau hygiénique ou l'article absorbant présente

une feuille supérieure perméable aux liquides,
une feuille arrière imperméable aux liquides jointe à la feuille supérieure, et
un absorbeur existant entre la feuille supérieure et la feuille arrière et incluant la résine absorbant l'eau.

**17.** Procédé de fabrication selon la revendication 16, dans lequel l'absorbeur inclut en outre un matériau de base en fibres.

**18.** Procédé de fabrication selon la revendication 16, dans lequel des particules de la résine absorbant l'eau sont fixées les unes aux autres par une colle dans l'absorbeur.

**19.** Procédé de fabrication selon la revendication 16 ou la revendication 17, dans lequel l'absorbeur inclus dans le matériau hygiénique ou l'article absorbant est une feuille de résine absorbant l'eau, et
la feuille de résine absorbant l'eau présente une structure multicouche dans laquelle la résine absorbant l'eau est prise en sandwich par des tissus non tissés.

[Fig. 1]

[Fig. 2]

[Fig. 3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1260361 A
- JP 9243622 A
- JP 2001194360 A
- US 7638570 B
- JP 10507967 W
- WO 9613335 A
- US 2003130638 A
- US 5102585 A
- WO 0033782 A
- JP 2010246990 A
- JP 2014534820 W

- JP 2017509393 W
- JP 2012034715 A
- JP 2009284984 A
- JP 2001555620 W
- JP 2013099429 A
- JP 6181948 A
- JP 2002360630 A
- JP 2001309945 A
- JP 2009291475 A
- JP 2016194387 A